# EUROPÄISCHE PATENTSCHRIFT

(11) **EP 1 368 320 B1**
(45) Veröffentlichungstag und Bekanntmachung des Hinweises auf die Patenterteilung: **16.08.2006**
(21) Anmeldenummer: 02722101.9
(22) Anmeldetag: 20.02.2002
(51) Int. Cl.: C07D 213/85, A61K 31/4418, C07D 405/04, C07D 417/12, C07D 409/12, A61K 31/443, A61K 31/4436, A61P 9/00

(54) **SUBSTITUIERTE 2-OXY-3,5-DICYANO-4-ARYL-6-AMINOPYRIDINE UND IHRE VERWENDUNG**
SUBSTITUTED 2-OXY-3,5-DICYANO-4-ARYL-6-AMINOPYRIDINES AND USE THEREOF
2-OXY-3,5-DICYANO-4-ARYL-6-AMINOPYRIDINES SUBSTITUEES ET LEUR UTILISATION

(30) Priorität: 05.03.2001 DE 10110438
(43) Veröffentlichungstag der Anmeldung: 10.12.2003
(73) Patentinhaber: Bayer HealthCare AG, 51368 Leverkusen (DE)
(72) Erfinder: ROSENTRETER, Ulrich, 42349 Wuppertal (DE); KRÄMER, Thomas, 42111 Wuppertal (DE); VAUPEL, Andrea, CH-4125 Riehen (CH); HÜBSCH, Walter, 42113 Wuppertal (DE); DIEDRICHS, Nicole, 42103 Wuppertal (DE); KRAHN, Thomas, 58135 Hagen (DE); DEMBOWSKY, Klaus, Boston, MA 02118 (US); STASCH, Johannes-Peter, 42651 Solingen (DE)
(86) Internationale Anmeldenummer: PCT/EP2002/001758
(87) Internationale Veröffentlichungsnummer: WO 2002/070484

(56) Entgegenhaltungen:
- WO-A-01/25210
- WO-A-01/62233
- WO-A-02/06237
- DATABASE CHEMCATS [Online] Chemical Abstracts Service, Columbus, Ohio, US ; XP002206424 & "Ambinter Exploratory Library" 21. Januar 2002 (2002-01-21) , AMBINTER , 75016 PARIS, FRANCE
- DATABASE CHEMCATS [Online] Chemical Abstracts Service, Columbus, Ohio, US; XP002206425 & "Otava Building Blocks" 2. Januar 2002 (2002-01-02) , OTAVA , 03187 KYIV-187, UKRAINE
- DATABASE CHEMCATS [Online] Chemical Abstracts Service, Columbus, Ohio, US; XP002206426 & "ChemBridge Product List" 17. Januar 2002 (2002-01-17) , CHEMBRIDGE CORPORATION , 92127 SAN DIEGO, US
- DATABASE CHEMCATS [Online] Chemical Abstracts Service, Columbus, Ohio, US; XP002206427 & "AsInEx Compound Collection" 10. Mai 2001 (2001-05-10) , ASINEX , 123182 MOSCOW, RUSSIA
- DATABASE CHEMCATS [Online] Chemical Abstracts Service, Columbus, Ohio, US; XP002206428 & "ChemDiv Inc. Product Library" 26. April 2001 (2001-04-26) , CHEMDIV INC. , 9212 SAN DIEGO, US
- DATABASE CHEMCATS [Online] Chemical Abstracts Service, Columbus, Ohio, US; XP002206429 & "Pharma Library Collection" 14. Mai 2001 (2001-05-14) , NANOSYN COMBINATORIAL SYNTHESIS INC. , 94043-2213 MOUNTAIN VIEW, US
- DATABASE CA [Online] CHEMICAL ABSTRACTS SERVICE, COLUMBUS, OHIO, US; QUINTELA, JOSE MARIA ET AL: "Reactivity of heterocyclic compounds. V. Behavior of 6-alkoxy-2-amino-(or chloro)-4-aryl-3,5-dicyanopyridines in the presence of nucleophiles" retrieved from STN Database accession no. 103:37345 XP002206430 & AN. QUIM., SER. C (1984), 80(3), 268-72 ,
- DATABASE CA [Online] CHEMICAL ABSTRACTS SERVICE, COLUMBUS, OHIO, US; QUINTELA, J. MARIA ET AL: "Reactivity of heterocyclic compounds. III. Behavior of 6-alkoxy-2-amino(or hydroxy)-3,5-dicyanopyridines toward electrophiles" retrieved from STN Database accession no. 102:6139 XP002206431 & AN. QUIM., SER. C (1983), 79(3), 368-72 ,
- FUENTES L ET AL: "AMALGAM (NA.HG) REDUCTION OF SOME 4-SUBSTITUTED-2-AMINO-3,5-DICYANO-6 -METHOXYPYRIDINES. NEW EVIDENCE REGARDING THE OXIDATION STEP IN THEIR SYNTHESIS" JOURNAL OF HETEROCYCLIC CHEMISTRY, PROVO, UT, US, Nr. 36, März 1999 (1999-03), Seiten 481-483, XP001080661 ISSN: 0022-152X
- SEADA, M. ET AL: "Reactions with 2-amino-3,5-dicyanopyridines" ORIENT. J. CHEM. (1989), 5(4), 273-80 , XP001087530 in der Anmeldung erwähnt
- CASTEDO, LUIS ET AL: "Synthesis and pharmacological activity of some nitrofuraldehyde cyanopyridine derivatives" EUR. J. MED. CHEM.--CHIM. THER. (1984), 19(6), 555-7 , XP000108754 in der Anmeldung erwähnt
- BALLANTYNE, BRYAN: "Acute toxicity and primary irritancy of 2-amino-3,5-dicyano-4-o- chlorophenyl-6-ethoxypyridine" DRUG CHEM. TOXICOL. (1977) (1985), 8(3), 171-82 , XP001087531 in der Anmeldung erwähnt
- QUINTELA J M ET AL: "A Ready One-pot Preparation for 7-Oxa(or thia)-3,4,6-triazabenz[d,e]a nthracene and 7-Oxa-3,4,6,9-tetrazabenz[d,e]anthracene Derivatives" TETRAHEDRON, ELSEVIER SCIENCE PUBLISHERS, AMSTERDAM, NL, Bd. 52, Nr. 31, 29. Juli 1996 (1996-07-29), Seiten 10497-10506, XP004104034 ISSN: 0040-4020 in der Anmeldung erwähnt
- MISHRIKY, N. ET AL: "New pyridinecarbonitriles from fluoro arylpropenones" RECL. TRAV. CHIM. PAYS-BAS (1994), 113(1), 35-9 , XP001088104 in der Anmeldung erwähnt
- CABRERIZO, M. ANGELES ET AL: "Synthesis of heterocycles. III. 2-Amino-3,5-dicyano-4-aryl-6- alkoxypyridines from benzylidenemalononitriles" AN. QUIM. (1974), 70(12), 951-8 , XP001088834
- ALVAREZ-INSUA, A. S. ET AL.: "Synthesis of Heterocaclic Compounds. II. A Simple One-Step Synthesis of Pyridines from Aldehydes and Malononitrile" JOURNAL OF HETEROCYCLIC CHEMISTRY, Bd. 7, 1970, Seiten 1305-1309, XP001088019
- POULSEN S-A ET AL: "ADENOSINE RECEPTORS: NEW OPPORTUNITIES FOR FUTURE DRUGS" BIOORGANIC & MEDICINAL CHEMISTRY, ELSEVIER SCIENCE LTD, GB, Bd. 6, 1998, Seiten 619-641, XP000985735 ISSN: 0968-0896 in der Anmeldung erwähnt
- BROADLEY, K. J.: "Drugs modulating adenosine receptors as potential therapeutic agents for cardiovascular diseases" EXPERT OPINIONS ON THERAPEUTIC PATENTS, Bd. 10, Nr. 11, 2000, Seiten 1669-1692, XP001084100 in der Anmeldung erwähnt

## Beschreibung

Die vorliegende Erfindung betrifft die Verwendung von substituierten 2-Oxy-3,5-dicyano-4-aryl-6-aminopyridine als Arzneimitteln sowie neue 2-Oxy-3,5-dicyano-4-aryl-6-aminopyridine und ein Verfahren zu ihrer Herstellung.

Adenosin, ein Nucleosid aus Adenin und D-Ribose, ist ein endogener Faktor mit zellprotektiver Wirksamkeit, insbesondere unter zellschädigenden Bedingungen mit begrenzter Sauerstoff- und Substratversorgung, wie z.B. bei Ischämie in verschiedensten Organen (z.B. Herz und Gehim).

Adenosin entsteht intrazellulär beim Abbau von Adenosin-5'-monophosphat (AMP) und S-Adenosylhomocystein als Zwischenprodukt, kann jedoch aus der Zelle freigesetzt werden und übt dann durch Bindung an spezifische Rezeptoren Funktionen als hormonähnliche Substanz oder Neurotransmitter aus.

Unter normoxischen Bedingungen ist die Konzentration des freien Adenosin im Extrazellulärraum sehr niedrig. Die extrazelluläre Konzentration von Adenosin erhöht sich in den betroffenen Organen jedoch dramatisch unter ischämischen bzw. hypoxischen Bedingungen. So ist beispielsweise bekannt, dass Adenosin die Thrombozyten-Aggregation hemmt und die Durchblutung der Herzkranzgefäße steigert. Weiterhin wirkt es auf die Herzfrequenz, auf die Ausschüttung von Neurotransmittern und auf die Lymphozyten-Differenzierung.

Diese Wirkungen von Adenosin zielen darauf ab, das Sauerstoffangebot der betroffenen Organe zu erhöhen bzw. den Stoffwechsel dieser Organe zu drosseln, um damit unter ischämischen oder hypoxischen Bedingungen eine Anpassung des Organstoffwechsels an die Organdurchblutung zu erreichen.

Die Wirkung von Adenosin wird über spezifische Rezeptoren vermittelt. Bekannt sind bisher die Subtypen A1, A2a, A2b und A3. Die Wirkungen dieser Adenosin-Rezeptoren werden intrazellulär durch den Botenstoff cAMP vermittelt. Im Falle der Bindung von Adenosin an die A2a- oder A2b-Rezeptoren kommt es über eine Aktivierung der membranständigen Adenylatzyklase zu einem Anstieg des intrazellulären cAMP, während die Bindung des Adenosin an die A1- oder A3-Rezeptoren über eine Hemmung der Adenylatzyklase eine Abnahme des intrazellulären cAMP-Gehalts bewirkt.

Als "Adenosinrezeptor-selektive Liganden" werden erfindungsgemäß solche Substanzen bezeichnet, die selektiv an einen oder mehrere Subtypen der Adenosinrezeptoren binden und dabei entweder die Wirkung des Adenosin nachahmen (Adenosin-Agonisten) oder dessen Wirkung blockieren (Adenosin-Antagonisten) können.

Adenosinrezeptor-selektive Liganden lassen sich nach ihrer Rezeptorselektivität in verschiedene Klassen einteilen, so z.B. in Liganden, die selektiv an die A1- oder die A2-Rezeptoren des Adenosin binden, bei letzteren auch beispielsweise solche, die selektiv an die A2a- oder die A2b-Rezeptoren des Adenosin binden. Auch sind Adenosinrezeptor-Liganden möglich, die selektiv an mehrere Subtypen der Adenosin-rezeptoren binden, so z.B. Liganden, die selektiv an die A1- und an die A2-, jedoch nicht an die A3-Rezeptoren des Adenosin binden.

Die zuvor genannte Rezeptor-Selektivität lässt sich bestimmen durch die Wirkung der Substanzen an Zelllinien, die nach stabiler Transfektion mit der entsprechenden cDNA die jeweiligen Rezeptorsubtypen exprimieren (siehe hierzu die Druckschrift M.E. Olah, H. Ren, J. Oströwski, K.A. Jacobson, G.L. Stiles, "Cloning, expression, and characterization of the unique bovine A1 adenosine receptor. Studies on the ligand binding site by site-directed mutagenesis." in J. Biol. Chem. 267 (1992) Seiten 10764-10770, deren Offenbarung hiermit im vollen Umfang durch Bezugnahme eingeschlossen ist).

Die Wirkung der Substanzen an solchen Zelllinien lässt sich erfassen durch biochemische Messung des intrazellulären Botenstoffes cAMP (siehe hierzu die Druckschrift K.N. Klotz, J. Hessling, J. Hegler, C. Owman, B. Kull, B.B. Fredholm, M.J. Lohse, "Comparative pharmacology of human adenosine receptor subtypes - characterization of stably transfected receptors in CHO cells" in Naunyn Schmiedebergs Arch. Pharmacol. 357 (1998) Seiten 1-9, deren Offenbarung hiermit im vollen Umfang durch Bezugnahme eingeschlossen ist).

Bei den aus dem Stand der Technik bekannten, als "adenosinrezeptor-spezifisch" geltenden Liganden handelt es sich überwiegend um Derivate auf Basis des natürlichen Adenosins (S.-A. Poulsen und R.J. Quinn, "Adenosine receptors: new opportunities for future drugs" in Bioorganic and Medicinal Chemistry 6 (1998) Seiten 619-641; K. J. Broadley, "Drugs modulating adenosine receptors as potential therapeutic agents for cardiovascular diseases" in Exp. Opin. Ther. Patents 10. (2000) Seiten 1669-1692). Die aus dem Stand der Technik bekannten Adenosin-Liganden haben jedoch meistens den Nachteil, dass sie nicht wirklich rezeptorspezifisch wirken, schwächer wirksam sind als das natürliche Adenosin oder nach oraler Applikation nur sehr schwach wirksam sind. Daher werden sie aufgrund der zuvor genannten Nachteile überwiegend nur für experimentelle Zwecke verwendet.

Aufgabe der vorliegenden Erfindung ist es, pharmakologisch aktive Substanzen aufzufinden oder bereitzustellen, die für die Prophylaxe und/oder Behandlung verschiedenster Erkrankungen, insbesondere Erkrankungen des Herz-KreislaufSystems (kardiovaskuläre Erkrankungen), geeignet sind und dabei vorzugsweise als Adenosinrezeptor-selektive Liganden wirken.

WO 02/06237 offenbart Dicyanopyridine und denen medizinische Verwendung.

Dicyanopyrimidine die gemäß der vorliegenden Erfindung zur Prophylaxe und/oder Behandlung von Erkrankungen verwendet werden können, sind teilweise literaturbekannt (siehe Alvarez-Insua, Lora-Tamayo, Soto, Journal of Heterocyclic Chemistry 7, Seiten 1305-1309, (1970); Quintela et al., Eur. J. Med. Chem. Chim. Ther. 19, 555-557 (1984); Ballantyne, Drug Chem. Toxicol. 8, 171-173 (1985); Seada et al., Orient. J. Chem. 5, 273-280, (1989); Mishriki et al., Recl. Trav. Chim. Pays-Bas 113, 35-39 (1994); Quintela et al., Tetrahedron 52, 10497-10506 (1996)). Jedoch ist in der Literatur für die bekannten Verbindungen eine therapeutische Anwendung bisher nicht beschrieben worden. Dies geschieht erstmals im Rahmen der vorliegenden Erfindung.

Die Verbindungen der Formel (I) können in Abhängigkeit vom Substitutionsmuster in stereoisomeren Formen, die sich entweder wie Bild und Spiegelbild (Enantiomere) oder die sich nicht wie Bild und Spiegelbild (Diastereomere) verhalten, existieren. Die Erfindung betrifft sowohl die Enantiomeren oder Diastereomeren als auch deren jeweilige Mischungen. Die Racemformen lassen sich ebenso wie die Diastereomeren in bekannter Weise in die stereoisomer einheitlichen Bestandteile trennen. Gleichermaßen betrifft die vorliegende Erfindung auch die Tautomeren der Verbindungen der Formel (I).

Salze der Verbindungen der Formel (1) können physiologisch unbedenkliche Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Trifluoressigsäure, Essigsäure, Propionsäure, Milchsäure, Weinsäure, Zitronensäure, Fumarsäure, Maleinsäure oder Benzoesäure.

Als Salze können auch Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabietylamin, 1-Ephenamin oder Methylpiperidin.

Als Hydrate bzw. Solvate werden erfindungsgemäß solche Formen der Verbindiingen der Formel (I) bezeichnet, welche in festem oder flüssigem Zustand durch Hydratation mit Wasser oder Koordination mit Lösungsmittelmolekülen eine Molekül-Verbindung bzw. einen Komplex bilden. Beispiele für Hydrate sind Sesquihydrate, Monohydrate, Dihydrate oder Trihydrate. Gleichermaßen kommen auch die Hydrate bzw. Solvate von Salzen der erfindungsgemäßen Verbindungen in Betracht.

Außerdem umfasst die Erfindung auch Prodrugs der erfindungsgemäßen Verbindungen. Als Prodrugs werden erfindungsgemäß solche Formen der Verbindungen der Formel (I) bezeichnet, welche selbst biologisch aktiv oder inaktiv sein können, jedoch unter physiologischen Bedingungen in die entsprechende biologisch aktive Form überführt werden können (beispielsweise metabolisch oder solvolytisch).

Im Rahmen der vorliegenden Erfindung haben die Substituenten, soweit nicht anders angegeben, die folgende Bedeutung:
Halogen steht im Allgemeinen für Fluor, Chlor, Brom oder Iod. Bevorzugt sind Fluor, Chlor oder Brom. Ganz besonders bevorzugt sind Fluor oder Chlor.
(C₁-C₈)-Alkyl,(C₁-C₆)-Alkyl bzw. (C₁-C₄)-Alkyl steht im Allgemeinen für einen geradkettigen oder verzweigten Alkylrest mit 1 bis 8, 1 bis 6 bzw. 1 bis 4 Kohlenstoffatomen. Bevorzugt ist ein geradkettiger oder verzweigter Alkylrest mit 1 bis 6 Kohlenstoffatomen. Besonders bevorzugt ist ein geradkettiger oder verzweigter Alkylrest mit 1 bis 4 Kohlenstoffatomen. Beispielsweise seien genannt: Methyl, Ethyl, n-Propyl, Isopropyl, n-Butyl, sec-Butyl, Isobutyl und tert.-Butyl.
(C₂-C₄)-Alkenyl stehen im Allgemeinen für einen geradkettigen oder verzweigten Alkenylrest mit 2 bis 4 Kohlenstoffatomen. Beispielsweise seien genannt: Vinyl, Allyl, Isopropenyl und n-But-2-en-1-yl.
(C₂-C₄)Alkinyl steht im Allgemeinen für einen geradkettigen oder verzweigten Alkinylrest mit 2 bis 4 Kohlenstoffatomen. Beispielsweise seien genannt: Ethinyl, n-Prop-2-in-1-yl und n-But-2-in-1-yl.
(C₁-C₈)-Alkoxy, (C₁-C₆)-Alkoxy bzw.(C₁-C₄)-Alkoxy steht im Allgemeinen für einen geradkettigen oder verzweigten Alkoxyrest mit 1 bis 8, 1 bis 6 bzw. 1 bis 4 Kohlenstoffatomen. Bevorzugt ist ein geradkettiger oder verzweigter Alkoxyrest mit 1 bis 6 Kohlenstoffatomen. Besonders bevorzugt ist ein geradkettiger oder verzweigter Alkoxyrest mit 1 bis 4 Kohlenstoffatomen. Beispielsweise seien genannt: Methoxy, Ethoxy, n-Propoxy, Isopropoxy, n-Butoxy, Isobutoxy, sec-Butoxy, tert.-Butoxy.
(C₁-C₄)-Alkoxycarbonyl steht im Allgemeinen für einen geradkettigen oder verzweigten Alkoxyrest mit 1 bis 4 Kohlenstoffatomen, der über eine Carbonylgruppe verknüpft ist. Beispielsweise seien genannt: Methoxycarbonyl, Ethoxycarbonyl, n-Propoxycarbonyl, Isopropoxycarbonyl und t-Butoxycarbonyl.
Mono- oder Di-(C₁-C₄)-alkylamino steht im Rahmen der Erfindung für eine AminoGruppe mit einem oder mit zwei gleichen oder verschiedenen geradkettigen oder verzweigten Alkylsubstituenten, die jeweils 1 bis 4 Kohlenstoffatome aufweisen. Beispielsweise seien genannt: Methylamino, Ethylamino, n-Propylamino, Isopropylamiho, t-Butylamino, *N,N*-Dimethylamino, *N,N*-Diethylamino, *N*-Ethyl-*N*-methylamino, *N*-Methyl-*N*-n-propylamino, *N*-Isopropyl-*N*-n-propylamino und *N*-t-Butyl-*N-*methylamino.
(C₃-C₇)-Cycloalkyl bzw. (C₃-C₆)-Cycloalkyl steht im Allgemeinen für einen cyclischen. Alkylrest mit 3 bis 7 bzw. 3 bis 6 Kohlenstoffatomen. Bevorzugt sind cyclische Alkylreste mit 3 bis 6 Kohlenstoffatomen. Beispielsweise seien genannt: Cyclopropyl, Cyclobutyl, Cyclopentyl oder Cyclohexyl.
(C₆-C₁₀)-Aryl steht im Allgemeinen für einen aromatischen Rest mit 6 bis 10 Kohlenstoffatomen. Bevorzugte Arylreste sind Phenyl und Naphthyl.
(C₆-C₁₀)-Aryloxy steht im Allgemeinen für einen wie zuvor definierten aromatischen Rest, der über ein Sauerstoffatom verknüpft ist.
5- bis 10-gliedriges Heteroaryl mit bis zu 3 Heteroatomen aus der Reihe N, O und/oder S steht im Allgemeinen für einen mono- oder bicyclischen, gegebenenfalls benzokondensierten Heteroaromaten, der über ein Ringkohlenstoffatom des Heteroaromaten, gegebenenfalls auch über ein Ringstickstoffatom des Heteroaromaten, verknüpft ist. Beispielsweise seien genannt: Pyridyl, Pyrimidyl, Pyridazinyl, Pyrazinyl, Thienyl, Furyl, Pyrrolyl, Pyrazolyl, Imidazolyl, Triazolyl, Thiazolyl, Oxazolyl, Oxdiazolyl, Isoxazolyl, Benzofuranyl, Benzothienyl oder Benzimidazolyl. Aus dieser Definition leiten sich analog die entsprechenden Heteroaromaten mit weniger Heteroatomen wie z.B. mit einem oder 2 Heteroatomen aus der Reihe N, O und/oder S oder geringerer Ringgröße wie z.B. 5- oder 6-gliedriges Heteroaryl ab. Im Allgemeinen gilt, dass 5-oder 6-gliedrige aromatische Heterocyclen mit einem oder 2 Heteroatomen aus der Reihe N, O und/oder S bevorzugt sind. Beispielsweise seien genannt: Pyridyl, Pyrimidyl, Pyridazinyl, Furyl, Imidazolyl oder Thienyl.
5- bis 7-gliedriger Heterocyclus steht im Allgemeinen für einen gesättigten oder teilweise ungesättigten, gegebenenfalls benzokondensierten Heterocyclus mit bis zu 3 Heteroatomen aus der Reihe N, O und/oder S. Beispielsweise seien genannt: Tetrahydrofuryl, Pyrrolidinyl, Pyrrolinyl, Dihydropyridinyl, Piperidinyl, Piperazinyl, Morpholinyl, Thiomorpholinyl, Hexahydropyranyl. Aus dieser Definition leiten sich analog die entsprechenden Heterocyclen mit weniger Heteroatomen wie z.B. mit einem oder 2 Heteroatomen aus der Reihe N, O und/oder S oder geringerer Ringgröße wie z.B. 5-oder 6-gliedriges Heterocyclyl ab.Bevorzugt sind gesättigte Heterocyclen mit bis zu 2 Heteroatomen aus der Reihe N, O und/oder S, insbesondere Piperidinyl, Piperazinyl, Morpholinyl und Pyrrolidinyl.

Gegenstand der vorliegenden Erfindung said neue Verbindungen der Formel (I) 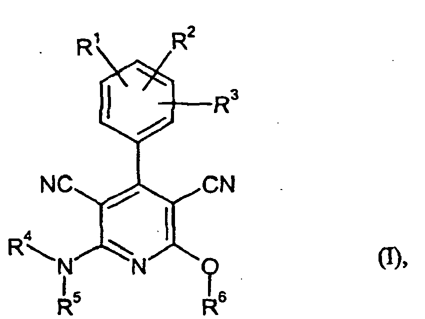 worin die Substituierten die im Anspruch 1 definierte Bedeutung besitzen.

Ein weiterer Gegenstand der Erfindung sind die folgenden erfindungsgemäßen Verbindungen:
2-(2-Hydroxyethylamino)-4-(1,3-benzodioxol-5-yl)-6-ethoxy-3,5-pyridindicarbonitril
2-(2-Methoxyethylamino)-4-(1,3-benzodioxol-5-yl)-6-ethoxy-3,5-pydridindicarbonitril
2-Amino-4-phenyl-6-(2-pyridinylmethoxy)-3,5-pydridindicarbonitril
2-Amino-4-phenyl-6-(3-pydridinylmethoxy)-3,5-pydridindicarbonitril
2-Amino-4-phenyl-6-(4-pyridinylmethoxy)-3,5-pyridindicarbonitril
2-Benzyloxy-6-(2-hydroxyethylamino)4-phenyl-3,5-pyridincarbonitril

Die vorliegende Erfindung betrifft weiterhin ein Verfahren zur Herstellung der Verbindungen der Formel (I), dadurch gekennzeichnet, dass man entweder
[A] Verbindungen der Formel (H) 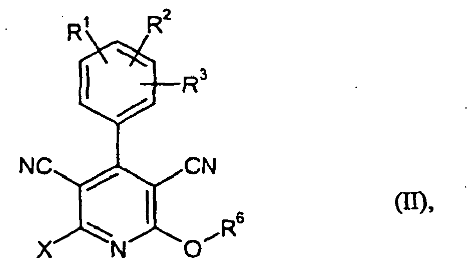 in welcher
   - R¹, R¹, R³ und R⁶: die oben angegebene Bedeutung haben und X für eine geeignete Abgangsgruppe, beispielsweise für Chlor, Brom, Methylthio oder Phenylthio steht,
   in einem inerten Lösungsmittel mit Verbindungen der Formel (III)

   R⁴ - NH - R⁵ (III),

   in welcher
   - R⁴ und R⁵: die oben angegebene Bedeutung haben,
   umsetzt
   oder
[B] Verbindungen der Formel (IV) 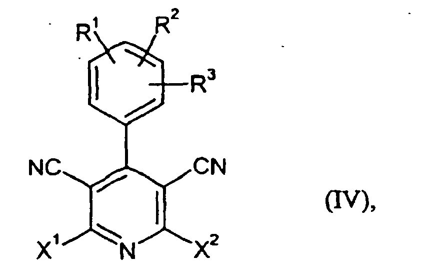 in welcher
   - R¹, R² und R³: die oben angegebene Bedeutung haben, und X¹ und X² unabhängig voneinander für geeignete Abgangsgruppen mit der zuvor definierten Bedeutung von X stehen,
   zunächst in einem inerten Lösungsmittel mit Verbindungen der Formel (III) in Verbindungen der Formel (V) 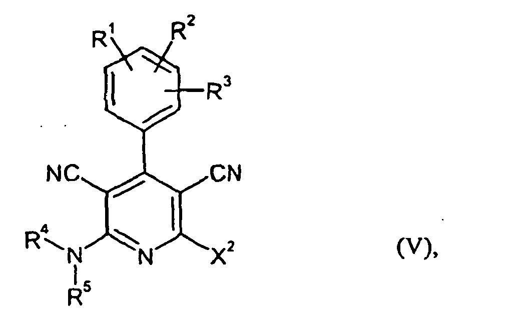 in welcher
   R¹, R², R³, R⁴, R⁵ und X² die oben angegebene Bedeutung haben,
   überführt und diese dann in Gegenwart einer Base, gegebenenfalls in einem inerten Lösungsmittel, mit Verbindungen der Formel (VI)

   R⁶-OH (VI),

   in welcher
   - R⁶: die oben angegebene Bedeutung hat,
   umsetzt
   oder
[C] für den Fall, dass in Verbindungen der Formel (I) R⁴ und R⁵ jeweils für Wasserstoff stehen, Verbindungen der Formel (VII) 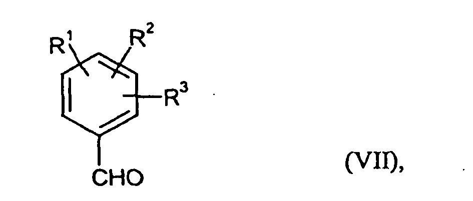
in welcher
R¹, R² und R³ die oben angegebene Bedeutung haben,
in Gegenwart einer Base, gegebenenfalls unter Zusatz eines inerten Lösungsmittels, mit Malodinitril und Verbindungen der Formel (VI) umsetzt.

Das erfindungsgemäße Verfahren kann durch das folgende Formelschema beispielhaft erläutert werden:

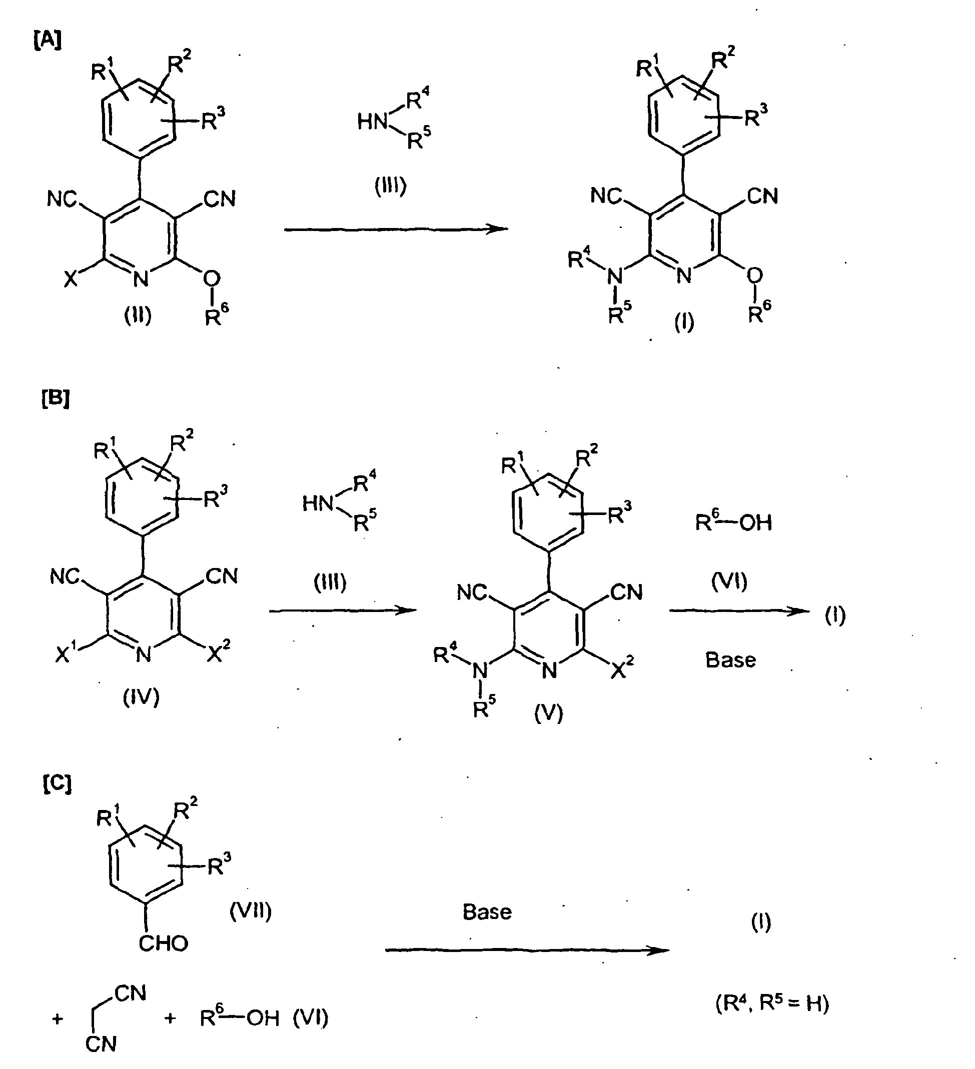

Für den Verfahrensschritt [A]: (II) + (III) → (I) eignen sich als Lösungsmittel organische Lösungsmittel, die sich unter den Reaktionsbedingungen nicht verändern. Hierzu gehören Alkohole wie Methanol, Ethanol und Isopropanoi, Ketone wie Aceton und Methylethylketon, acyclische und cyclische Ether wie Diethylether und Tetrahydrofuran, Ester wie Essigsäureethylester oder Essigsäurebutylester, Kohlenwasserstoffe wie Benzol, Xylol, Toluol, Hexan oder Cyclohexan, chlorierte Kohlenwasserstoffe wie Dichlormethan, Chlorbenzol oder Dichlorethan oder andere Lösungsmittel wie Dimethylformamid, Acetonitril, Pyridin oder Dimethylsulfoxid (DMSO). Wasser ist als Lösungsmittel ebenso geeignet. Ebenso ist es möglich, Gemische der zuvor genannten Lösungsmittel einzusetzen. Bevorzugt ist Tetrahydrofuran.

Die Reaktion erfolgt im Allgemeinen in einem Temperaturbereich von -78°C bis +150°C, bevorzugt im Bereich von +20°C bis +80°C, insbesondere bei +20°C bis +40°C.

Die Umsetzung kann bei normalem, erhöhtem oder erniedrigtem Druck durchgeführt werden, z.B. im Bereich von 0,5 bis 5 bar. Im Allgemeinen arbeitet man bei Normaldruck.

Die Umsetzung erfolgt im Allgemeinen mit einem Überschuss an Verbindung (III), bevorzugt in einem Verhältnis von 2 bis 8 Mol der Verbindung (III) zu 1 Mol der Verbindung (II).

Die Verbindungen der allgemeinen Formel (II) sind dem Fachmann bekannt oder können in Analogie zu literaturbekannten Methoden hergestellt werden [siehe beispielsweise J.M. Quintela, J.L. Soto, Anales de Quimica 79, 368-372 (1983)].

Die Verbindungen der allgemeinen Formel (III) sind kommerziell erhältlich, dem Fachmann bekannt oder nach literaturüblichen Methoden herstellbar.

Für den ersten Verfahrensschritt [B]: (IV) + (III) → (V) eignen sich als Lösungsmittel organische Lösungsmittel, die unter den Reaktionsbedingungen inert sind. Hierzu gehören Ketone wie Aceton und Methylethylketon, acyclische und cyclische Ether wie Diethylether, 1,2-Dimethoxyethan oder Tetrahydrofuran, Ester wie Essigsäureethylester oder Essigsäure-butylester, Kohlenwasserstoffe wie Benzol, Xylol, Toluol, Hexan oder Cyclohexan, chlorierte Kohlenwasserstoffe wie Dichlormethan, Chlorbenzol oder Dichlorethan oder andere Lösungsmittel wie Dimethylformamid, Acetonitril, Pyridin oder Dimethylsulfoxid (DMSO). Ebenso ist es möglich, Gemische der zuvor genannten Lösungsmittel einzusetzen. Bevorzugt sind 1,2-Dimethoxyethan oder Tetrahydrofuran.

Die Reaktion erfolgt im Allgemeinen in einem Temperaturbereich von -78°C bis +120°C, bevorzugt im Bereich von +20°C bis +60°C, insbesondere bei +20°C bis +40°C.

Die Umsetzung kann bei normalem, erhöhtem oder erniedrigtem Druck durchgefiihrt werden, z.B. im Bereich von 0,5 bis 5 bar. Im Allgemeinen arbeitet man bei Normaldruck.

Die Umsetzung erfolgt im Allgemeinen mit einer äquivalenten Menge oder mit einem Überschuss an Verbindung (III), bevorzugt in einem Verhältnis von 1 bis 8 Mol der Verbindung (III), besonders bevorzugt in einem Verhältnis von 1 bis 2 Mol der Verbindung (III) zu 1 Mol der Verbindung (IV).

Die Umsetzung kann auch in Gegenwart von Hilfsbasen wie beispielsweise Trialkylaminen wie Triethylamin oder Düsopropylethylamin, Alkalicarbonaten wie Natrium- oder Kaliumcarbonat oder Amidinen wie DBN (1,5-Diazabicyclo[4.3.0]non-5-en) oder DBU (1,8-Diazabicyclo[5.4.0]undec-7-en) durchgefiihrt werden.

Die Verbindungen der allgemeinen Formel (IV) sind bekannt oder können in Analogie zu literaturbekannten Methoden hergestellt werden [siehe beispielsweise Quintela et al., Heterocycles 38, 1299-1305 (1994)].

Die Verbindungen der allgemeinen Formel (V) sind bekannt oder können in Analogie zu literaturbekannten Methoden hergestellt werden [siehe beispielsweise Quintela et al., Heterocycles 38, 1299-1305 (1994), Kambe et al., Synthesis, 531-533 (1981), Elnagdi et al., Z. Naturforsch. 47b, 572-578 (1991)].

Der zweite Verfahrensschritt [B]: (V) + (VI) → (I) kann ohne Lösungsmittel in Substanz oder in einem Lösungsmittel stattfinden. Geeignet als Lösungsmittel sind organische Lösungsmittel, die unter den Reaktionsbedingungen inert sind. Hierzu gehören Ketone wie Aceton und Methylethylketon, acyclische und cyclische Ether wie Diethylether, 1,2-Dimethoxyethan oder Tetrahydrofuran, Ester wie Essigsäureethylester oder Essigsäure-butylester, Kohlenwasserstoffe wie Benzol, Xylol, Toluol, Hexan oder Cyclohexan, chlorierte Kohlenwasserstoffe wie Dichlormethan, Chlorbenzol oder Dichlorethan oder andere Lösungsmittel wie Dimethylformamid, Acetonitril, Pyridin oder Dimethylsulfoxid (DMSO). Wasser ist als Lösungsmittel ebenso geeignet. Ebenso ist es möglich, Gemische der zuvor genannten Lösungsmittel einzusetzen. Bevorzugt sind Acetonitril, 1,2-Dimethoxyethan und Tetrahydrofuran.

Als Basen eignen sich die üblichen anorganischen oder organischen Basen. Hierzu gehören Alkalihydroxide wie beispielsweise Natrium- oder Kaliumhydroxid, Alkalicarbonate wie Natrium- oder Kaliumcarbonat oder Natrium- oder Kaliumhydrogencarbonat, Kalium-tert.-butylat, Natriumhydrid, Amide wie Natriumamid, Lithium- bis-(trimethylsilyl)amid oder Lithiumdiisopropylamid, metallorganische Verbindungen wie Butyllithium oder Phenyl-lithium, Amine wie Triethylamin oder Pyridin, oder auch das Natrium- oder Kaliumsalz der jeweiligen Verbindung der allgemeinen Formel (VI) selbst. Bevorzugt sind Kalium-tert.-butylat und Kaliumcarbonat.

Die Base kann hierbei in einem Verhältnis von 1 bis 10 Mol, bevorzugt in einem Verhältnis von 1 bis 5 Mol, insbesondere in einem Verhältnis von 1 bis 4 Mol Base zu 1 Mol der Verbindung (VI) eingesetzt werden.

Die Reaktion erfolgt im Allgemeinen in einem Temperaturbereich von -78°C bis +120°C, bevorzugt im Bereich von +20°C bis +100°C, insbesondere bei +20°C bis +80°C.

Die Umsetzung kann bei normalem, erhöhtem oder erniedrigtem Druck durchgeführt werden, z.B. im Bereich von 0,5 bis 5 bar. Im Allgemeinen arbeitet man bei Normaldruck.

Die Umsetzung erfolgt im Allgemeinen mit einer äquivalenten Menge oder mit einem Überschuss an Verbindung (VI), bevorzugt in einem Verhältnis von 1 bis 50 Mol der Verbindung (VI) zu 1 Mol der Verbindung (V).

Die Verbindungen der allgemeinen Formel (VI) sind kommerziell erhältlich, dem Fachmann bekannt oder nach literaturüblichen Methoden herstellbar.

Verfahren [C]: (VII) --> (I), wobei R⁴= R⁵ = H, wird in Analogie zu einer literaturbekannten Methode durchgeführt [Alvarez-Insua et al., Journal of Heterocyclic Chemistry 7, 1305-1309 (1970)].

Die Umsetzung kann ohne Lösungsmittel in Substanz oder in einem Lösungsmittel stattfinden. Geeignet als Lösungsmittel sind organische Lösungsmittel, die unter den Reaktionsbedingungen inert sind. Hierzu gehören Ketone wie Aceton und Methylethylketon, acyclische und cyclische Ether wie Diethylether, 1,2-Dimethoxyethan oder Tetrahydrofuran, Ester wie Essigsäureethylester oder Essigsäurebutylester, Kohlenwasserstoffe wie Benzol, Xylol, Toluol, Hexan oder Cyclohexan, chlorierte Kohlenwasserstoffe wie Dichlormethan, Chlorbenzol oder Dichlorethan oder andere Lösungsmittel wie Dimethylformamid, Acetonitril, Pyridin oder Dimethylsulfoxid (DMSO). Ebenso ist es möglich, Gemische der zuvor genannten Lösungsmittel einzusetzen. Bevorzugt ist die Umsetzung ohne Lösungsmittel in Substanz.

Als Basen eignen sich die üblichen anorganischen oder organischen Basen. Hierzu gehören Alkalihydroxide wie beispielsweise Natrium- oder Kaliumhydroxid, Alkalicarbonate wie Natrium- oder Kaliumcarbonat oder Natrium- oder Kaliumhydrogencarbonat, Kalium-tert.-butylat, Natriumhydrid, Amide wie Natriumamid, Lithium- bis-(trimethylsilyl)amid oder Lithiumdiisopropylamid, metallorganische Verbindungen wie Butyllithium oder Phenyl-lithium, Amine wie Triethylamin oder Pyridin, oder auch das Natrium- oder Kaliumsalz der jeweiligen Verbindung der allgemeinen Formel (VI) selbst. Bevorzugt ist das Natrium- oder Kaliumsalz der jeweiligen Verbindung der allgemeinen Formel (VI).

Die Base kann hierbei in einem Verhältnis von 1 bis 10 Mol, bevorzugt in einem Verhältnis von 1 bis 5 Mol, insbesondere in einem Verhältnis von 1 bis 4 Mol Base zu 1 Mol der Verbindung (VII) eingesetzt werden.

Die Reaktion erfolgt im Allgemeinen in einem Temperaturbereich von -78°C bis +180°C, bevorzugt im Bereich von +20°C bis +160°C, insbesondere bei +20°C bis +120°C.

Die Umsetzung kann bei normalem, erhöhtem oder erniedrigtem Druck durchgeführt werden z.B. im Bereich von 0,5 bis 5 bar. Im Allgemeinen arbeitet man bei Normaldruck.

Die Umsetzung erfolgt im Allgemeinen mit einer äquivalenten Menge oder mit einem Überschuss an Verbindung (VI), bevorzugt in einem Verhältnis von 1 bis 50 Mol (VI) zu 1 Mol der Verbindung (VII).

Die Verbindungen der allgemeinen Formel (VII) sind kommerziell erhältlich, dem Fachmann bekannt oder nach literaturüblichen Methoden herstellbar.

Überraschenderweise zeigen die Verbindungen der Formel (I) ein nicht vorhersehbares pharmakologisches Wirkspektrum und sind daher insbesondere zur Prophylaxe und/oder Behandlung von Erkrankungen geeignet.

Die Verbindungen der Formel (I) sind zur Prophylaxe und/oder Behandlung einer ganzen Reihe von Erkrankungen geeignet, so beispielsweise insbesondere von Erkrankungen des Herzkreislaufsystems (kardiovaskulären Erkrankungen).

Im Sinne der vorliegenden Erfindung sind unter Erkrankungen des Herz-KreislaufSystems bzw. kardiovaskulären Erkrankungen beispielsweise insbesondere die folgenden Erkrankungen zu verstehen: Koronare Herzkrankheit, Hypertonie (Bluthochdruck), Restenose wie z.B. Restenose nach Ballondilatation von peripheren Blutgefäßen, Arteriosklerose, Tachykardien, Arrhythmien, periphere und kardiale Gefäßerkrankungen, stabile und instabile Angina pectoris und Vorhofflimmern.

Weiterhin eignen sich die Verbindungen der Formel (I) beispielsweise insbesondere auch zur Reduktion des von einem Infarkt betroffenen Myokardbereichs.

Des Weiteren eignen sich die Verbindungen der Formel (I) beispielsweise insbesondere zur Prophylaxe und/oder Behandlung von thromboembolischen Erkrankungen und Ischämien wie Myokardinfarkt, Himschlag und transitorischen ischämischen Attacken.

Weitere Indikationsgebiete, für das sich die Verbindungen der Formel (I) eignen, sind beispielsweise insbesondere die Prophylaxe und/oder Behandlung von Erkrankungen des Urogenitalbereiches, wie z.B. Reizblase, erektile Dysfunktion und weibliche sexuelle Dysfunktion, und Krebs, daneben aber auch die Prophylaxe und/oder Behandlung von inflammatorischen Erkrankungen, wie z.B. Asthma und entzündlichen Dermatosen, von neuroinflammatorischen Erkrankungen des Zentralnervensystems, wie beispielsweise Zustände nach Himinfarkt, der Alzheimer-Erkrankung, weiterhin auch von neurodegenerative Erkrankungen wie der Parkinson-Erkrankung, sowie von Schmerzzuständen.

Ein weiteres Indikationsgebiet sind beispielsweise insbesondere die Prophylaxe und/oder Behandlung von Erkrankungen der Atemwege wie beispielsweise Asthma, chronische Bronchitis, Lungenemphysem, Bronchiektasen, zystische Fibrose (Mukoviszidose) und pulmonale Hypertonie.

Des Weiteren kommen die Verbindungen der Formel (I) auch beispielsweise insbesondere für die Prophylaxe und/oder Behandlung von Leberfibrose und Leberzirrhose in Betracht.

Schließlich kommen die Verbindungen der Formel (I) beispielsweise insbesondere auch für die Prophylaxe und/oder Behandlung von Diabetes, insbesondere Diabetes mellitus, in Betracht.

Die vorliegende Erfindung betrifft auch die Verwendung der Substanzen der Formel (I) zur Herstellung von Arzneimitteln und pharmazeutischen Zusammensetzungen zur Prophylaxe und/oder Behandlung der zuvor genannten Krankheitsbilder.

Die vorliegende Erfindung betrifft weiterhin ein Verfahren zur Prophylaxe und/oder Behandlung der zuvor genannten Krankheitsbilder mit den Substanzen der Formel (I).

Die pharmazeutische Wirksamkeit der zuvor genannten Verbindungen der Formel (I) lässt sich durch ihre Wirkung als selektive Liganden an einzelnen oder mehreren Subtypen der Adenosin-Rezeptoren, insbesondere als selektive Liganden an Adenosin-A1-, Adenosin-A2a- und/oder Adenosin-A2b-Rezeptoren, vorzugsweise als selektive Liganden an Adenosin-A1- und/oder Adenosin-A2b-Rezeptoren erklären.

Als "selektiv" werden im Rahmen der vorliegenden Erfindung solche Adenosinrezeptor-Liganden bezeichnet, bei denen einerseits eine deutliche Wirkung an einem der mehreren Adenosin-Rezeptor-Subtypen und andererseits keine oder eine deutlich schwächere Wirkung an einem oder mehreren anderen Adenosin-Rezeptor-Subtypen zu beobachten ist, wobei bezüglich der Testmethoden für die Wirk-Selektivität Bezug genommen wird auf die im Abschnitt A. II. beschriebenen Testmethoden.

Ein Vorteil der erfindungsgemäßen Verbindungen der Formel (I) ist, dass sie gegenüber Adenosinrezeptor-Liganden des Standes der Technik selektiver wirken.

Insbesondere wirken Verbindungen der Formel (I), worin R¹ und R² an benachbarte Phenylringatome gebunden sind und für eine Gruppe -O-CH₂-O- , -O-CH₂-CH₂-O-oder -O-(CH₂)₃-O- stehen, im Allgemeinen selektiv an Adenosin-A1-Rezeptoren.

Insbesondere wirken Verbindungen der Formel (I), worin einer der Reste R¹, R² oder R³ für -NH-C(O)- R⁷ steht und einer der Reste R⁴ oder R⁵ für Benzyl oder Pyridylmethyl steht, im Allgemeinen selektiv an Adenosin-A1- und Adenosin-A2b-Rezeptoren.

Die Rezeptorselektivität kann bestimmt werden durch die biochemische Messung des intrazellulären Botenstoffes cAMP in den transfizierten Zellen, die spezifisch nur einen Subtyp der Adenosinrezeptoren exprimieren. Im Falle von A2a- bzw. A2b-Agonisten (Kopplung bevorzugt über Gs-Proteine) wird dabei ein Anstieg des intrazellulären cAMP-Gehaltes, im Falle von A2a- bzw.A2b-Antagonisten eine Abnahme des intrazellulären cAMP-Gehaltes nach Vorstimulation mit Adenosin oder Adenosin ähnlichen Substanzen beobachtet (siehe Druckschriften B. Kull, G. Arslan, C. Nilsson, C. Owman, A. Lorenzen, U. Schwabe, B.B. Fredholm, "Differences in the order of potency for agonists but not antagonists at human and rat adenosine A2A receptors", Biochem. Pharmacol., 57 (1999) Seiten 65-75; und S.P. Alexander, J. Cooper, J. Shine, S.J. Hill, "Characterization of the human brain putative A2B adenosine receptor expressed in Chinese hamster ovary (CHO.A2B4) cells", Br. J. Pharmacol., 119 (1996) Seiten 1286-90, deren jeweilige Offenbarung hiermit durch Bezugnahme eingeschlossen ist). Entsprechend führen A1-Agonisten (Kopplung bevorzugt über Gi-Proteine) zu einer Abnahme und A1-Antagonisten zu einem Anstieg im cAMP-Gehalt.

So eignen sich Verbindungen der Formel (I), die selektiv an Adenosin-Al-Rezeptoren binden, bevorzugt zur Myokard-Protektion und zur Prophylaxe und/oder Behandlung von Tachykardien, Vorhof Arrhythmien, Herzinsuffizienz, Heizinfarkt, akutem Nierenversagen, Diabetes, Schmerzzuständen sowie zur Wundheilung.

Verbindungen der Formel (I), die selektiv an Adenosin-A2a-Rezeptoren binden, sind bevorzugt zur Prophylaxe und/oder Behandlung von thrombo-embolischen Erkrankungen, von neurodegenerativen Erkrankungen wie Morbus Parkinson sowie zur Wundheilung geeignet.

Verbindungen der Formel (I), die selektiv an Adenosin-A2b-Rezeptoren binden, eignen sich bevorzugt zur Prophylaxe und/oder Therapie der Leberfibrose, des Herzinfarkts, von neuroinflammatorischen Erkrankungen, der Alzheimer-Erkrankung, von urogenitaler Inkontinenz sowie von Atemwegserkrankungen wie beispielsweise Asthma und chronischer Bronchitis.

Weiterer Gegenstand der vorliegenden Erfindung sind Arzneimittel und pharmazeutische Zusammensetzungen, die mindestens eine Verbindung der Formel (I), vorzugsweise zusammen mit einem oder mehreren pharmakologisch unbedenklichen Hilfs- oder Trägerstoffen enthalten, sowie deren Verwendung zu den zuvor genannten Zwecken.

Für die Applikation der Verbindungen der Formel (I) kommen alle üblichen Applikationsformen in Betracht, d.h. also oral, parenteral, inhalativ, nasal, sublingual, rektal, lokal, wie z.B. bei Implantaten oder Stents, oder äußerlich wie z.B. transdermal. Bei der parenteralen Applikation sind insbesondere intravenöse, intramuskuläre, subkutane Applikation zu nennen, z.B. als subkutanes Depot. Besonders bevorzugt ist die orale Applikation.

Hierbei können die Wirkstoffe allein oder in Form von Zubereitungen verabreicht werden. Für die orale Applikation eignen sich als Zubereitungen u.a. Tabletten, Kapseln, Pellets, Dragees, Pillen, Granulate, feste und flüssige Aerosole, Sirupe, Emulsionen, Suspensionen und Lösungen: Hierbei muss der Wirkstoff in einer solchen Menge vorliegen, dass eine therapeutische Wirkung erzielt wird. Im Allgemeinen kann der Wirkstoff in einer Konzentration von 0,1 bis 100 Gew.-%, insbesondere 0,5 bis 90 Gew.-%, vorzugsweise 5 bis 80 Gew.%, vorliegen, d.h. der Wirkstoff sollte in Mengen vorliegen, die ausreichend sind, den angegebenen Dosierungsspielraum zu erreichen.

Zu diesem Zweck können die Wirkstoffe in an sich bekannter Weise in die üblichen Zubereitungen überführt werden. Dies geschieht unter Verwendung inerter, nichttoxischer, pharmazeutisch geeigneter Trägerstoffe, Hilfsstoffe, Lösungsmittel, Vehikel, Emulgatoren und/oder Dispergiermittel.

Als Hilfsstoffe seien beispielsweise aufgeführt: Wasser, nichttoxische organische Lösungsmittel wie z.B. Paraffine, pflanzliche Öle (z.B. Sesamöl), Alkohole (z.B, Ethanol, Glycerin), Glykole (z.B. Polyethylenglykol), feste Trägerstoffe wie natürliche oder synthetische Gesteinsmehle (z.B. Talkum oder Silikate), Zucker (z.B. Milchzucker), Emulgiermittel, Dispergiermittel (z.B. Polyvinylpyrrolidon) und Gleitmittel (z.B. Magnesiumsulfat).

Im Falle der oralen Applikation können Tabletten auch allgemein übliche Zusätze wie Natriumcitrat zusammen mit Zuschlagstoffen wie Stärke, Gelatine und dergleichen enthalten. Wässrige Zubereitungen für die orale Applikation können weiterhin mit Geschmacksaufbesserem oder Farbstoffen versetzt werden.

Im Allgemeinen hat es sich als vorteilhaft erwiesen, bei parenteraler Applikation Mengen von etwa 0,1 bis etwa 10 000 µg/kg, vorzugsweise etwa 1 bis etwa 1 000 µg/kg, insbesondere etwa 1 µg/kg bis etwa 100 µg/kg Körpergewicht, zur Erzielung wirksamer Ergebnisse zu verabreichen. Bei oraler Applikation beträgt die Menge etwa 0,1 bis etwa 10 mg/kg, vorzugsweise etwa 0,5 bis etwa 5 mg/kg, insbesondere etwa 1 bis etwa 4 mg/kg Körpergewicht.

In Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt, kann es gegebenenfalls erforderlich sein, von den genannten Mengen abzuweichen.

Die vorliegende Erfindung wird an den folgenden Beispielen veranschaulicht, die die Erfindung jedoch keinesfalls beschränken.

### A. Bewertung der physiologischen Wirksamkeit

### I. Nachweis der kardiovaskulären Wirkung

### Langendorff-Herz der Ratte:

Narkotisierten Ratten wird nach Eröffnung des Brustkorbes das Herz entnommen und in eine konventionelle Langendorff-Apparatur eingeführt. Die Koronararterien werden volumenkonstant (10 ml/min) perfundiert und der dabei auftretende Perfusionsdruck wird über einen entsprechenden Druckaufnehmer registriert. Eine Abnahme des Perfusionsdrucks in dieser Anordnung entspricht einer Relaxation der Koronararterien. Gleichzeitig wird über einen in die linke Herzkammer eingeführten Ballon und einen weiteren Druckaufnehmer der Druck gemessen, der vom Herzen während jeder Kontraktion entwickelt wird. Die Frequenz des isoliert schlagenden Herzens wird rechnerisch aus der Anzahl der Kontraktionen pro Zeiteinheit ermittelt.

### II. Nachweis der Rezeptorselektivität

### a) Adenosin-A1-, A2a-, A2b- und A3-Rezeptorselektivität

Zellen der permanenten Linie CHO (Chinese Hamster Ovary) werden stabil mit der cDNA für die Adenosin-Rezeptor-Subtypen A1, A2a, A2b und A3 transfiziert. Die Bindung der Substanzen an die A2a- oder A2b-Rezeptorsubtypen wird bestimmt durch Messung des intrazellulären cAMP-Gehaltes in diesen Zellen mit einem konventionellen radioimmunologischen Assay (cAMP-RIA).

Im Falle der Wirkung der Substanzen als Agonisten kommt es als Ausdruck der Bindung der Substanzen zu einem Anstieg des intrazellulären cAMP-Gehaltes. Als Referenzverbindung dient in diesen Experimenten die Adenosin-analoge Verbindung NECA (5-N-Ethylcarboxamido-adenosin), die nicht selektiv, aber mit hoher Affinität an alle Adenosin-Rezeptor-Subtypen bindet und eine agonistische Wirkung besitzt (Klotz, K.N., Hessling, J., Hegler, J., Owman, C., Kull, B., Fredholm, B.B., Lohse, M.J., Comparative pharmacology of human adenosine receptor subtypes - characterization of stably transfected receptors in CHO cells, Naunyn Schmiedebergs Arch Pharmacol, 357 (1998), 1-9).

Die Adenosin-Rezeptoren A1 und A3 sind an ein Gi-Protein gekoppelt, d.h. eine Stimulation dieser Rezeptoren führt zu einer Inhibition der Adenylatcyclase und somit zu einer Senkung des intrazellulären cAMP-Spiegels. Zur Identifizierung von A1/A3-Rezeptor-Agonisten wird die Adenylatcyclase mit Forskolin stimuliert. Eine zusätzliche Stimulation der A1/A3-Rezeptoren hemmt jedoch die Adenylatcyclase, so dass A1/A3-Rezeptor-Agonisten über einen vergleichsweise geringen Gehalt der Zelle an cAMP detektiert werden können.

Für den Nachweis einer antagonistischen Wirkung an Adenosin-Rezeptoren werden die mit dem entsprechenden Rezeptor transfizierten, rekombinanten Zellen mit NECA vorstimuliert und die Wirkung der Substanzen auf eine Reduktion des intrazellulären cAMP-Gehalts durch diese Vorstimulation untersucht. Als Referenzverbindung dient in diesen Experimenten XAC (xanthine amine congener), die nicht selektiv, aber mit hoher Affinität an alle Adenosinrezeptor-Subtypen bindet und eine antagonistische Wirkung besitzt (Müller, C.E., Stein, B., Adenosine receptor antagonists: structures and potential therapeutic applications, Current Pharmaceutical Design, 2 (1996), 501-530).

### b) Adenosin-A1-, A2a-, A2b- Rezeptorselektivität

Zellen der permanenten Linie CHO (Chinese Hamster Ovary) werden stabil mit der cDNA für die Adenosin-Rezeptor-Subtypen A1, A2a, A2b transfiziert. Die Adenosin A1 Rezeptoren sind über Gᵢ-Proteine und die Adenosin A2a und A2b Rezeptoren über Gs-Froteine an die Adenylatcyclase gekoppelt. Entsprechend wird die cAMP-Bildung in der Zelle inhibiert bzw. stimuliert. Über einen cAMP-abhängigen Promotor wird danach die Expression der Luziferase moduliert. Der Luciferase-Test wird mit dem Ziel hoher Sensitivität und Reproduzierbarkeit, geringer Varianz und guter Eignung für die Durchführung auf einem Robotersystem optimiert durch Variation mehrerer Testparameter, wie z.B. Zelldichte, Dauer der Anzuchtphase und der Testinkubation, Forskolin-Konzentration, Medium-Zusammensetzung. Zur pharmakologischen Charakterisierung der Zellen und zum Roboter-gestützten Substanztest-Screening wird das folgende Testprotokoll verwendet:

Die Stammkulturen wird in DMEM/F12 Medium mit 10% FCS (fötales Kälberserum) bei 37°C unter 5 % CO₂ gezüchtet und jeweils nach 2-3 Tagen 1:10 gesplittet. Testkulturen werden von 1000 bis 3000 Zellen pro Napf in 384-well Platten ausgesät und ca. 48 Stunden bei 37°C angezogen. Dann wird das Medium durch eine physiologische Kochsalzlösung (130 mM NaCl, 5 mM KCl, 2 mM CaCl₂, 20 mM HEPES, 1 mM MgCl₂•6H₂O,5 mM NaHCO₃, pH 7,4) ersetzt. Die in DMSO gelösten Substanzen werden 3 mal 1:10 mit dieser physiologischen Kochsalzlösung verdünnt und zu den Testkulturen pipettiert (maximale DMSO-Endkonzentration im Testansatz: 0,5%) So erhält man Substanzendkonzentrationen von beispielsweise 5 µM bis 5 nM. 10 Minuten später wird Forskolin zu den A1 Zellen zugegeben und anschließend werden alle Kulturen für 4 Stunden bei 37°C inkubiert. Danach wird zu den Testkulturen 35 µl Lösung, bestehend zu 50 % aus Lysereagenz (30 mM di-Natriumhydrogenphosphat, 10 % Glycerin, 3 % TritonX100, 25 mM TrisHCl, 2 mM Dithiotreitol (DTT), pH 7,8) und zu 50% aus Luciferase Substrat Lösung (2,5 mM ATP, 0,5 mM Luciferin, 0,1 mM Coenzym A, 10 mM Tricin, 1,35 mM MgSO₄, 15 mM DTT, pH 7,8) zugegeben, ca. 1 Minute geschüttelt und die Luciferase-Aktivität mit einem Kamerasystem gemessen.

### B. Ausführungsbeispiele

### Verwendete Abkürzungen:

- DMSO: Dimethylsulfoxid
- d.Th.: der Theorie
- HPLC: Hochdruck-, Hochleistungsflüssigchromatographie
- NMR: Kernresonanzspektroskopie
- THF: Tetrahydrofuran
- i. V.: im Vakuum

### Beispiel 1

### 2-Amino-4-(1,3-benzodioxol-5-yl)-6-benzyloxy-3,5-pyridindicarbonitril

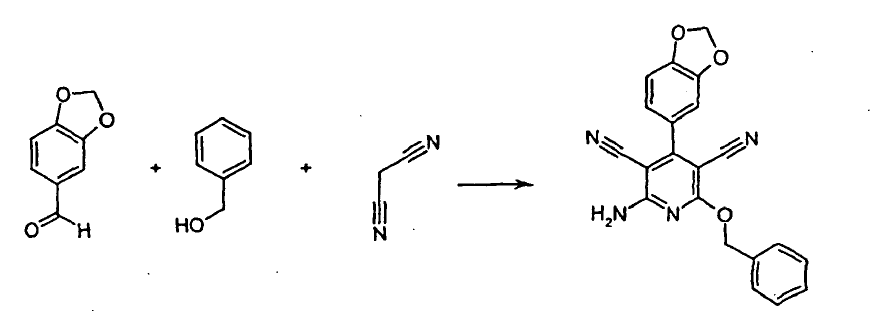

344 mg (15 mmol) Natrium werden in 20,7 ml Benzylalkohol gelöst. Dann werden 660 mg (10 mmol) Malodinitril und 750 mg (5 mmol) Piperönal zugegeben und ca. 16 h bei Raumtemperatur gerührt. Die Reaktionsmischung wird mit 20 ml Wasser versetzt und mit 1N Salzsäure neutral gestellt. Es wird dreimal mit je 50 ml Dichlormethan extrahiert, die vereinigten organischen Phasen werden mit Natriumsulfat getrocknet und i. V. eingedampft. Der Eindampfrückstand wird durch Chromatographie an Kieselgel (Dichlormethan als Elutionsmittel) gereinigt.
Ausbeute: 872 mg (= 40,1 % d. Th.)
Massenspektrum: gesuchte Molmasse: 370, gefunden [M+H]⁺ = 371
NMR-Spektrum: [¹H-NMR, DMSO-d₆] 5,45 [2H] s; 6,15 [2H] s; 7,0 - 7,2 [3H] m; 7,3 - 7,6 [5H] m; 7,8 - 8,2 [2H] s breit.

### Beispiel 2

### 2-Amino-4-(1,3-benzodioxol-5-yl)-6-ethoxy-3,5-pyridindicarbonitril

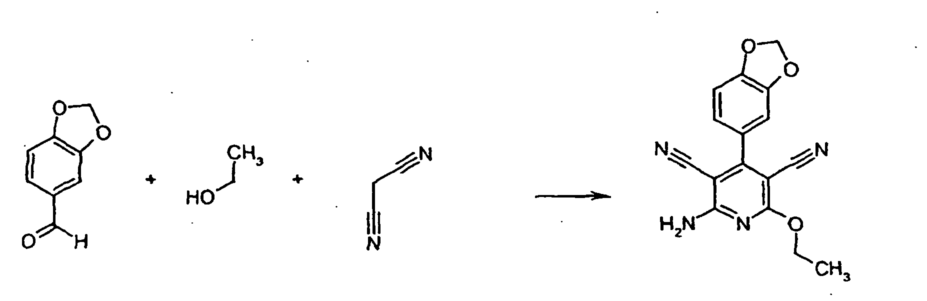

Die Verbindung wird analog zu Beispiel 1 hergestellt.
Ausbeute: 850 mg (= 41,4 % d. Th.)
Massenspektrum: gesuchte Molmasse: 308, gefunden [M+H]⁺ = 309

### Beispiel 3

### 2-Amino-4-(1,3-benzodioxol-5-yl)-6-(2-hydroxyethoxy)-3,5-pyridindicarbonitril

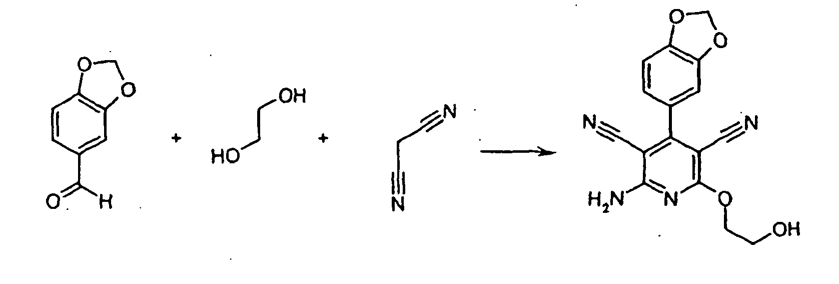

Die Verbindung wird analog zu Beispiel 1 hergestellt.
Ausbeute: 1100 mg (= 50,9 % d. Th.)
Massenspektrum: gesuchte Molmasse: 324, gefunden [M+H]⁺ = 325

### Beispiel 4

### 2-Ethylamino-4-(1,3-benzodioxol-5-yl)-6-benzyloxy-3,5-pyridindicarbonitril

### 1. Stufe:

### 2-Chlor-4-(1,3-benzodioxol-5-yl)-6-benzyloxy-3,5-pyridindicarbonitril

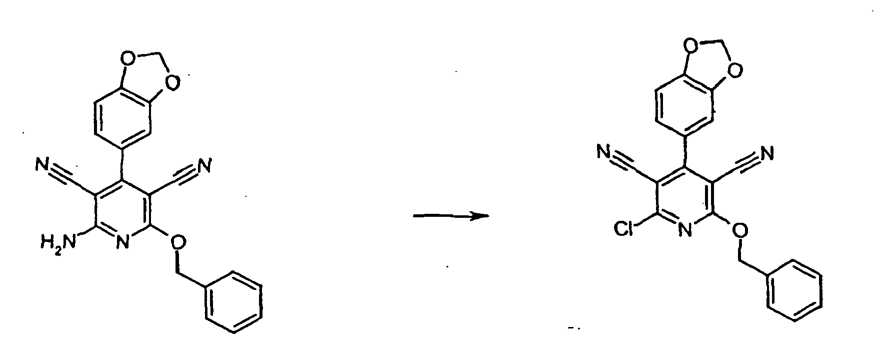

400 mg (1,08 mmol) 2-Amino-4-(1,3-benzodioxol-5-yl)-6-benzyloxy-3,5-pyridindicarbonitril (Beispiel I) werden in 10 ml Acetonitril gelöst. Dann werden 759 mg (0,87 ml, 6,48 mmol) Isoamylnitrit und 871 mg (6,48 mmol) Kupfer(I)chforid zugegeben und ca. 16 h bei 40°C gerührt. Die Reaktionslösung wird dann mit 1N Salzsäure verdünnt und dreimal mit je 50 ml Dichlormethan extrahiert. Die vereinigten organischen Phasen werden mit Natriumsulfat getrocknet und i. V. eingedampft. Der Eindampfrückstand wird aus Dichlormethan/Methanol kristallisiert.
Ausbeute: 214 mg (= 50,9 % d. Th.)
Massenspektrum: gesuchte Molmasse: 389, gefunden [M+H]⁺ = 390

### 2. Stufe:

### 2-Ethylamino-4-(1,3-benzodioxol-5-yl)-6-benzyloxy-3,5-pyridindicarbonitril

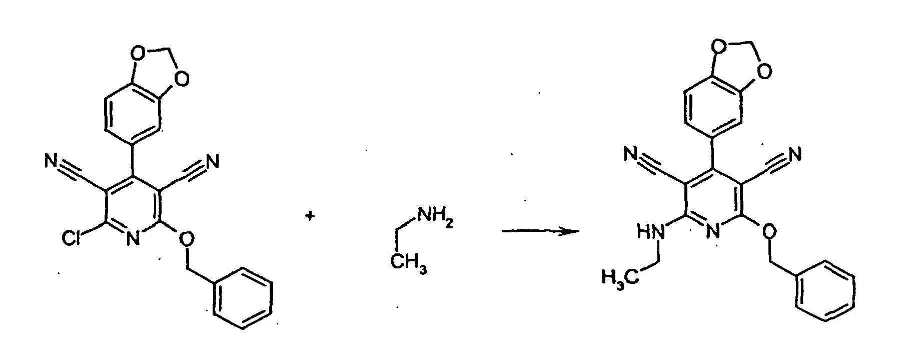

30 mg (0,08 mmol) 2-Chlor-4-(1,3-benzodioxol-5-yl)-6-benzyloxy-3,5-pyridindicarbonitril (Beispiel 4, 1. Stufe) werden zusammen mit 10,4 mg (0,23 mmol) Ethylamin in 0,12 ml THF ca. 16 h geschüttelt. Die Reaktionslösung wird mit Wasser versetzt, das auskristallisierte Produkt abgesaugt und i. V. getrocknet.
Ausbeute: 22 mg (= 72,7 % d. Th.)
Massenspektrum: gesuchte Molmasse: 398, gefunden [M+H]⁺ = 399

### Beispiel 5

### 2-(2-Hydroxyethylamino)-4-(1,3-benzodioxol-5-yl)-6-benzyloxy-3,5-pyridindicarbonitril

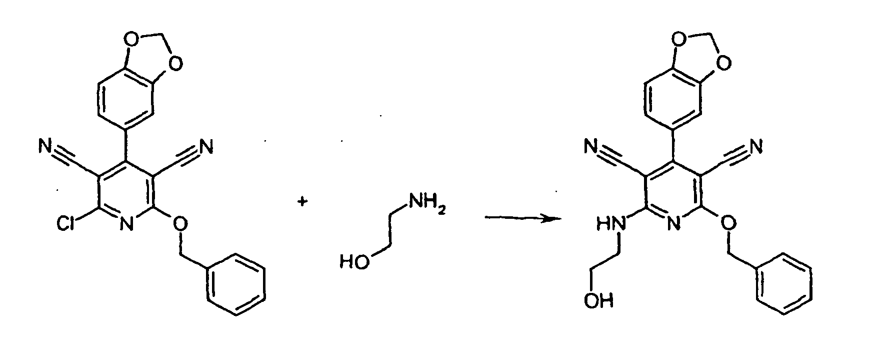

30 mg (0,08 mmol) 2-Chlor-4-(1,3-benzodioxol-5-yl)-6-benzyloxy-3,5-pyridindicarbonitril (Beispiel 4, 1. Stufe) werden zusammen mit 14,1 mg (0,23 mmol) 2-Hydroxyethylamin in 0,5 ml THF ca. 16 h geschüttelt. Die Reaktionslösung wird mit Wasser versetzt, das auskristallisierte Produkt abgesaugt und i.V. getrocknet. Das Rohprodukt wird durch Chromatographie an Kieselgel mit Dichlormethan und Dichlormethan/Methanol 50:1 als Eluens gereinigt.
Ausbeute: 16 mg (= 50 % d. Th.)
Massenspektrum: gesuchte.Molmasse: 414, gefunden [M+H]⁺= 415
NMR-Spektrum: [¹H-NMR, DMSO-d₆] 3,55 [4H] s; 4,8 [1H] s; 5,5 [2H] s; 6,15 [2H] s; 7,0 - 7,2 [3H] m; 7,3-7,6 [5H] m; 8 [1H] s.

### Beispiel 6

### 2-Methylamino-4-(1,3-benzodioxol-S-yl)-6-benzyloxy-3,5-pyridindicarbonitril

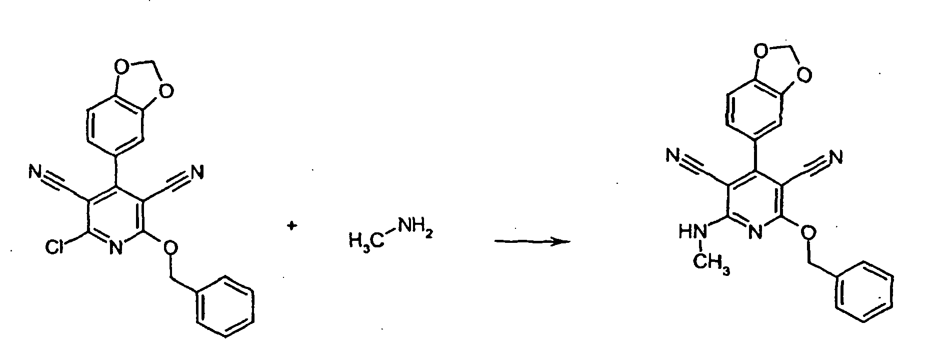

Die Verbindung wird analog zu Beispiel 4, 2. Stufe hergestellt.
Ausbeute: 26 mg (= 88,9 % d. Th.)
Massenspektrum: gesuchte Molmasse: 384, gefunden [M+H]⁺ = 385

### Beispiel 7

### 2-(2-Methoxyethylamino)-4-(1,3-benzodioxol-5-yl)-6-benzyloxy-3,5-pyridindicarbonitril

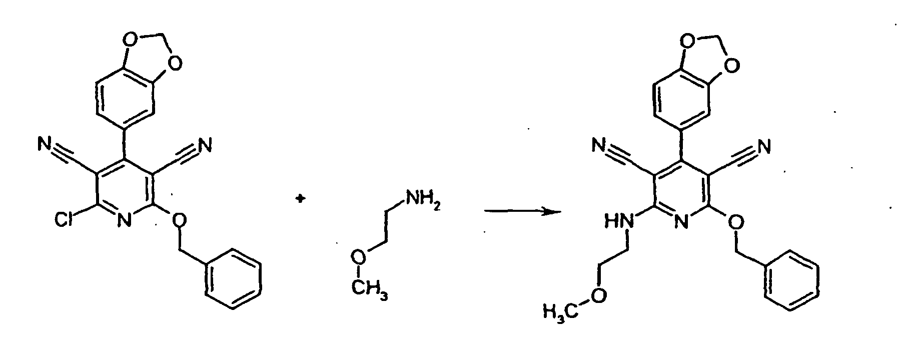

Die Verbindung wird analog zu Beispiel 4, 2. Stufe hergestellt.
Ausbeute: 27 mg (= 82,8 % d. Th.)
Massenspektrum: gesuchte Molmasse: 428, gefunden [M+H]⁺= 429

### Beispiel 8

### 2-Cyclopropylamino-4-(1,3-benzodioxol-5-yl)-6-benzyloxy-3,5-pyridindicarbonitril

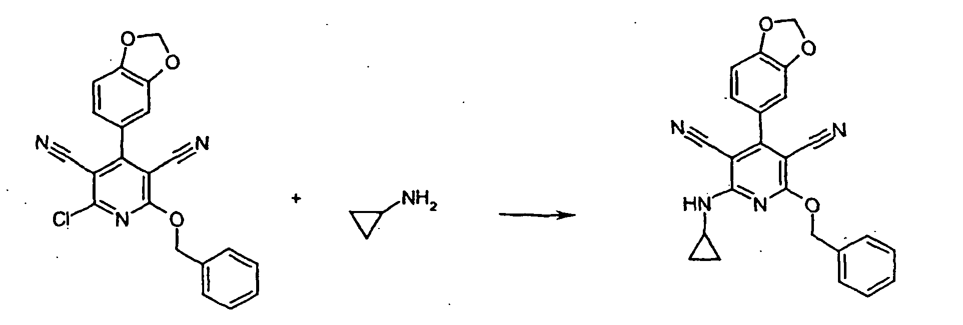

Die Verbindung wird analog zu Beispiel 4, 2. Stufe hergestellt.
Ausbeute: 24 mg (= 76,9 % d. Th.)
Massenspektrum: gesuchte Molmasse: 410, gefunden [M+H]⁺= 411

### Beispiel 9

### 2-Cyclopropylmethylamino-4-(1,3-benzodioxol-5-yl)-6-benzyloxy-3,5-pyridindicarbonitril

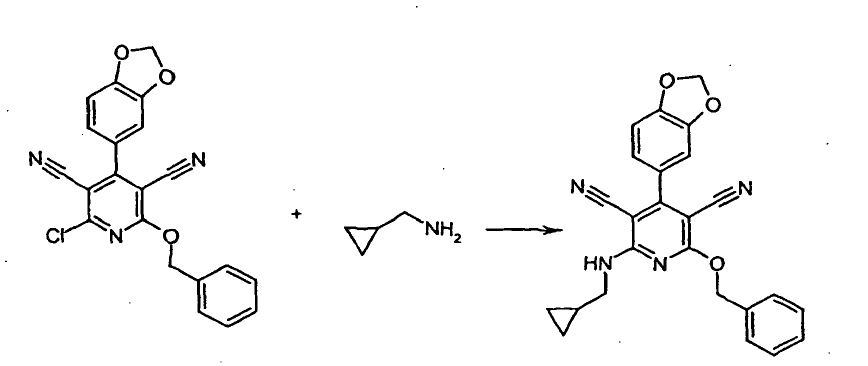

Die Verbindung wird analog zu Beispiel 4, 2. Stufe hergestellt.
Ausbeute: 24 mg (= 73,5 % d. Th.)
Massenspektrum: gesuchte Molmasse: 424, gefunden [M+H]⁺ = 425

### Beispiel 10

### 2-(2-Hydroxyethylamino)-4-(1,3-benzodioxol-5-yl)-6-ethoxy-3,5-pyridindicarbonitril

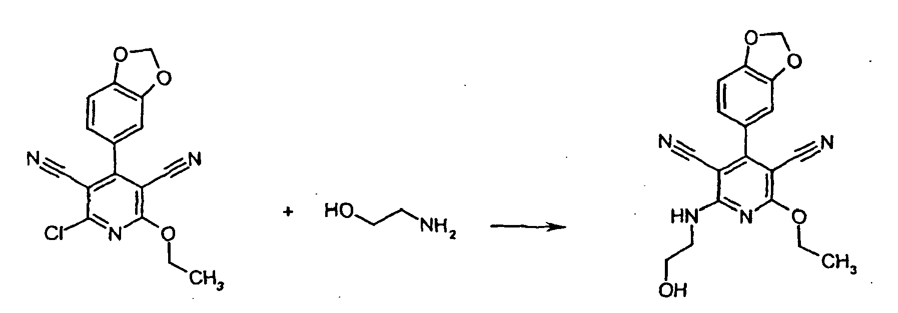

Das Edukt 2-Chlor-4-(1,3-benzodioxol-5-yl)-6-ethoxy-3,5-pyridindicarbonitril wird analog zu Beispiel 4 - 1. Stufe hergestellt, das Produkt 2-(2-Hydroxyethylamino)-4-(1,3-benzodioxol-5-yl)-6-ethoxy-3,5-pyridindicarbonitril wird analog zu Beispiel 4, 2. Stufe hergestellt.
Ausbeute: 22 mg (= 39,9 % d. Th.)
Massenspektrum: gesuchte Molmasse: 352, gefunden [M+H]⁺ = 353

### Beispiel 11

### 2-Cyclopropylamino-4-(1,3-benzodioxol-5-yl)-6-(2-hydroxyethoxy)-3,5-pyridindicarbonitril

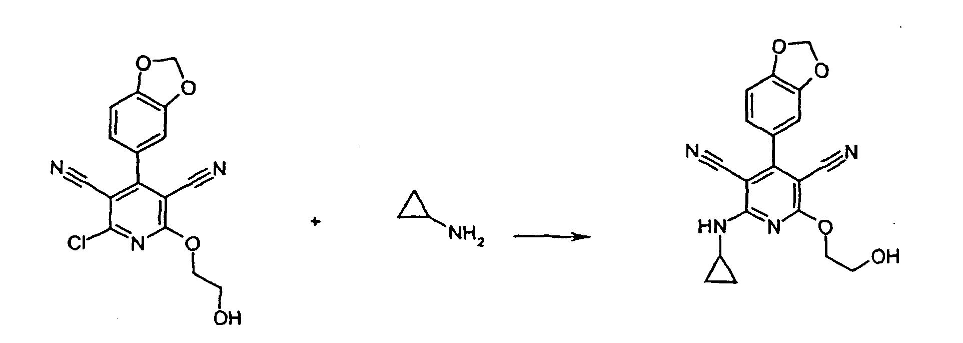

Das Edukt 2-Chlor-4-(1,3-ben2odioxol-5-yl)-6-(2-hydroxyethoxy)-3,5-pyridin-dicarbonitril wird analog zu Beispiel 4-1. Stufe hergestellt, das Produkt 2-Cyclopropylamino-4-(1,3-benzodioxol-5-yl)-6-(2-hydroxyethoxy)-3,5-pyridindicarbonitril wird analog zu Beispiel 4, 2. Stufe hergestellt.
Ausbeute: 51 mg (= 80 % d. Th.)
Massenspektrum: gesuchte Molmasse: 364, gefunden [M+H]⁺ = 365

### Beispiel 12

### 2-(2-Methoxyethylamino)-4-(1,3-benzodioxol-5-yl)-6-ethoxy-3,5-pyridindicarbonitril

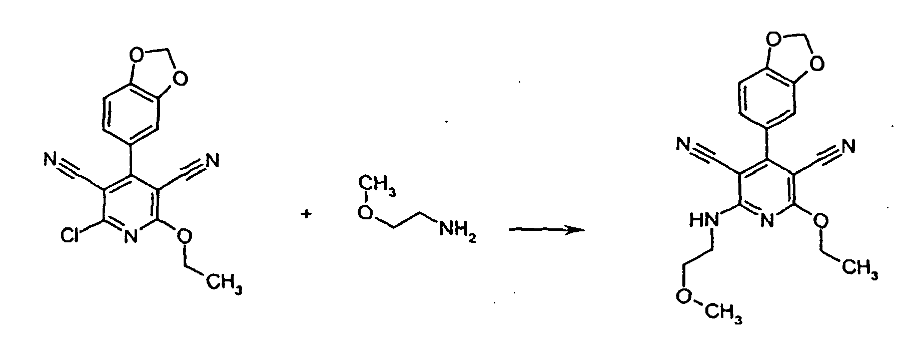

Das Edukt 2-Chlor-4-(1,3-benzodioxol-5-yl)-6-ethoxy-3,5-pyridindicarbonitril wird analog zu Beispiel 4 - 1. Stufe hergestellt, das Produkt 2-(2-Methoxyethylamino)-4-(1,3-benzodioxol-5-yl)-6-ethoxy-3,5-pyridindicarbonitril wird analog zu Beispiel 4, 2. Stufe hergestellt.
Ausbeute: 54 mg (= 96,5 % d. Th.)
Massenspektrum: gesuchte Molmasse: 366, gefunden [M+H]⁺= 367

### Beispiel 13

### 2-Amino-4-phenyl-6-(2-pyridi nylmethoxy)-3,5-pyridindicarbonitril

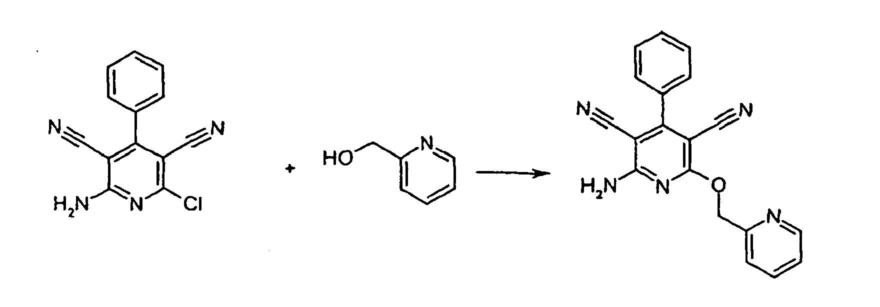

102 mg (0,4 mmol) 2-Amino-6-chlor-4-phenyl-3,5-pyridindicarbonitril [Qumteta et al., Heterocycles 38, 1299-1305 (1994)] werden zusammen mit 54 mg (0,48 mmol) Kalium tert.butylat in 1,696 g (=1,5 ml, 15,55 mmol) 2-Hydroxymethylpyridin gelöst und ca. 16 h bei 60°C gerührt. Das auskristallisierte Produkt wird abgesaugt, mit Wasser gewaschen und i. V. getrocknet.
Ausbeute: 128 mg (= 97,8 % d. Th.)
Massenspektrum: gesuchte Molmasse: 327, gefunden [M+H]⁺ = 328
NMR-Spektrum: [¹H-NMR, DMSO-d₆] 5,55 [2H] s; 7,35 [1H] m; 7,5 [6H] m; 7,85 [1H] m; 8,05 [2H] s breit; 8,6 [1H] m.

### Beispiel 14

### 2-Amino-4-phenyl-6-(3-pyridinylmethoxy)-3,5-pyridindicarbonitril

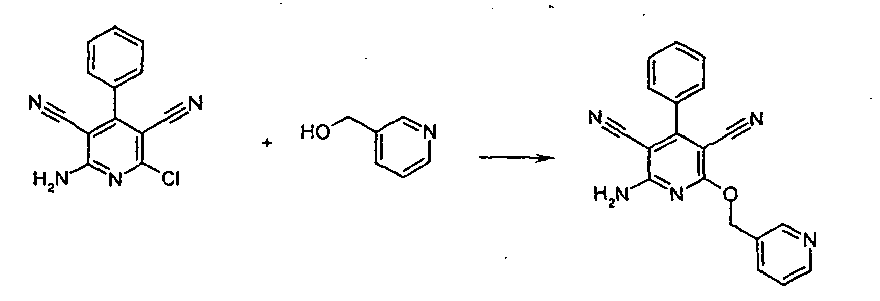

102 mg (0,4 mmol) 2-Amino-6-chlor-4-phenyl-3,5-pyridindicarbonitril [Quintela et al., Heterocycles 38, 1299-1305 (1994)] werden zusammen mit 54 mg (0,48 mmol) Kalium tert.butylat in 1,696 g (=1,5 ml, 15,55 mmol) 3-Hydroxymethylpyridin gelöst und ca. 16 h bei 60°C gerührt. Die entstandene Suspension wird mit zwei Tropfen Eisessig angesäuert. Das auskristallisierte Produkt wird abgesaugt, mit Wasser gewaschen und i. V. getrocknet.
Ausbeute: 130 mg (= 99,7 % d. Th.)
Massenspektrum: gesuchte Molmasse: 327, gefunden [M+H]⁺= 328
NMR-Spektrum: [¹H-NMR, DMSO-d₆] 5,5 [2H] s; 7,45 [6H] m; 7,95 [1H] m; 8,1 [2H] s breit; 8,55 [1H] m; 8,8 [1H] d.

### Beispiel 15

### 2-Amino-4-phenyl-6-(4-pyridinylmethoxy)-3,5-pyridindicarbonitril

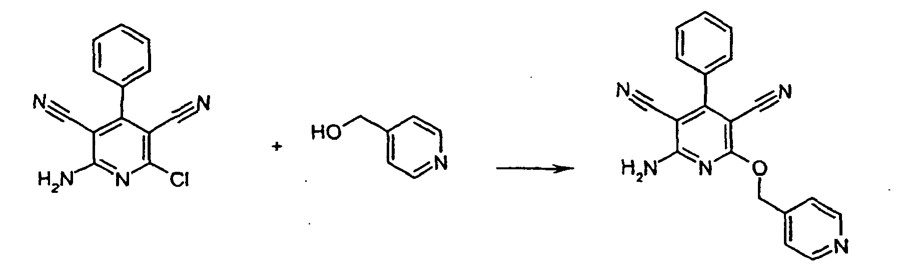

102 mg (0,4 mmol) 2-Amino-6-chlor-4-phenyl-3,5-pyridindicarbonitril [Quintela et al., Heterocycles 38, 1299-1305 (1994)] werden zusammen mit 54 mg (0,48 mmol) Kalium tert.butylat und 131 mg (1,2 mmol) 4-Hydroxymethylpyridin gelöst in 1,5 ml DMSO und ca. 16 h bei 60°C gerührt. Das Reaktionsgemisch wird mit zwei Tropfen Eisessig angesäuert. Die Lösung wird durch präparative HPLC an Reversed Phase Kieselgel gereinigt (Gradient: Wasser + 0,1 % Ameisensäure / Acetonitril 90:10 ⇒ 10:90 in 12 Minuten).
Ausbeute: 66 mg (= 50,5 % d. Th.)
Massenspektrum: gesuchte Molmasse: 327, gefunden [M+H]⁺= 328

### Beispiel 16

### N- (4-[2-amino-6-(benzyloxy)-3,5-dieyano-4-pyridinyllphenyl) acetainid

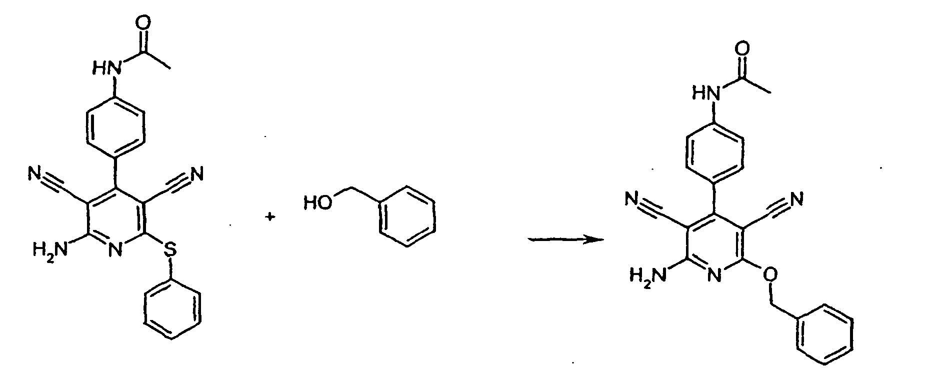

58 mg (0,15 mmol) N-{4-[2-Amino-3,5-dicyano-6-(phenylsulfanyl)-4-pyridinyl]-phenyl}acetamid [hergestellt analog Kambe et al., Synthesis, 53I-533 (1981)] werden zusammen mit 41 mg (0,3 mmol) Kaliumcarbonat in 1,88 g (=1,8 ml, 17,4 mmol) Benzylalkohol gelöst/suspendiert und 3,5 h bei 100°C gerührt. Der Benzylalkohol wird i.V. abgedampft, der Eindampfrückstand wird durch präparative HPLC an Reversed Phase Kieselgel gereinigt (Gradient: Wasser / Acetonitril 90:10 ⇒ 10:90 in 38 Minuten).
Ausbeute: 38 mg (= 67 % d. Th.)
Massenspektrum: gesuchte Molmasse: 383, gefunden [M+H]⁺ = 384

### Beispiel 17

### 2-Benzyloxy-6-(2-hydroxyethylamino)-4-phenyl-3,5-pyridindicarbonitril

### 1. Stufe:

### 2-Chlor-4-phenyl-6-(2-hydroxyethylamino)-3,5-pyridindicarbonitril

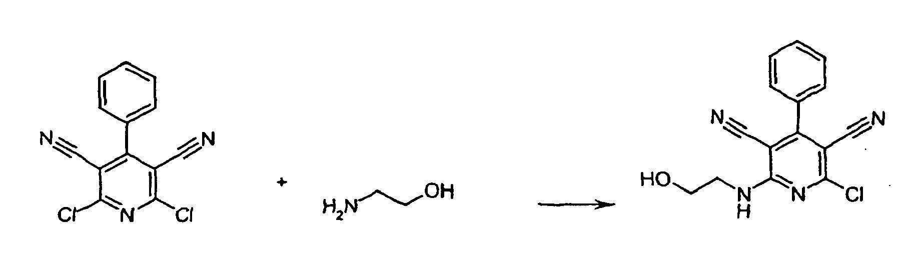

1 g (3,65 mmol) 2,6-Dichlor-4-phenyl-3,5-pyridindicarbonitril [Quintela et al., Heterocycles 38, 1299-1305 (1994)] werden zusammen mit 0,33 g (5,47 mmol) 2-Aminoethanol in 3 ml THF gelöst und 8 h unter Rückfluss erhitzt. Das Reaktionsgemisch wird durch präparative HPLC an Reversed Phase Kieselgel gereinigt (Gradient: Wasser + 0,1 % Ameisensäure/Acetonitril 90:10 => 5:95 in 35 Minuten).
Ausbeute: 977 mg (= 89,7 % d. Th.)
Massenspektrum: gesuchte Molmasse: 298, gefunden [M+H]⁺ = 299

### 2. Stufe:

### 2-Benzyloxy-6-(2-hydroxyethylamino)-4-phenyl-3,5-pyridindicarbonitril

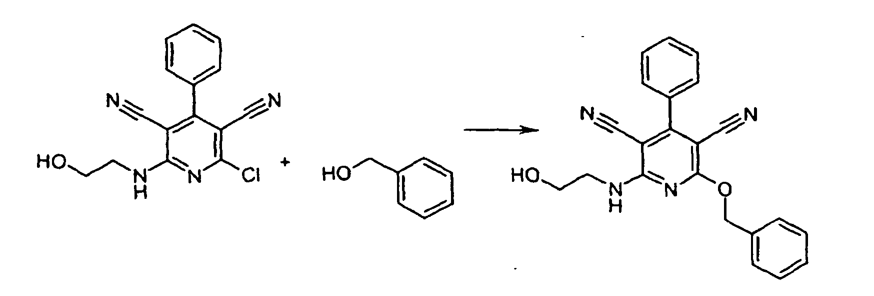

58 mg (0,2 mmol) 2-Chlor-4-phenyl-6-(2-hydroxyethylamino)-3,5-pyridindicarbonitril (Beispiel 17, 1. Stufe) werden zusammen mit 27 mg (0,24 mmol) Kalium tert.butylat in 0,96 g (=1 ml, 8,9 mmol) Benzylalkohol gelöst und ca. 16 h bei 40°C gerührt. Das Reaktionsgemisch wird durch präparative HPLC an Reversed Phase Kieselgel gereinigt (Gradient: Wasser + 0,1 % Ameisensäure/Acetonitril 90:10 ⇒ 5:95 in 35 Minuten).
Ausbeute: 62 mg (= 83,3 % d. Th.)
Massenspektrum: gesuchte Molmasse: 370, gefunden [M+H]⁺= 371

### Beispiel 18

### N- {4-[2-amino-6-(3-pyridylmethyloxy)-3,5-dicyano-4-pyridinyl]phenyl} acetamid

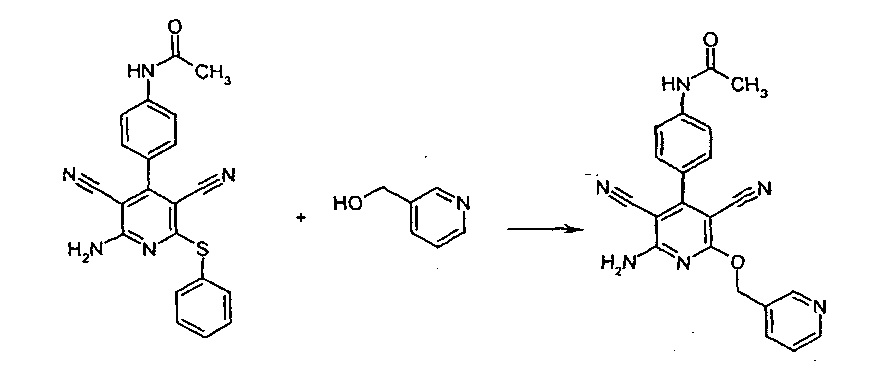

115 mg (0,3 mmol) N-{4-[2-Amino-3,5-dicyano-6-(phenylsulfanyl)-4-pyridinyl]-phenyl}acetamid [hergestellt analog Kambe et al., Synthesis, 531-533 (1981)] werden zusammen mit 50 mg (0,45 mmol) Kalium tert.butylat in 1,12 g (= 1 ml, 10,3 mmol) 3-Pyridylmethylalkohol gelöst/suspendiert und 2 h bei 60°C gerührt. Der Niederschlag wird abgesaugt, in einem Gemisch aus Wasser und Ethanol suspendiert und mit 0,3 ml 5N Essigsäure versetzt. Nach Filtration wird durch präparative HPLC an Reversed Phase Kieselgel gereinigt (Gradient: Wasser / Acetonitril 95:5 ⇒10:90 in 38 Minuten).
Ausbeute: 24 mg (= 20 % d. Th.)
Massenspektrum: gesuchte Molmasse: 384, gefunden [M+H]⁺ = 385
NMP,-Spektrum: [¹H-NMR, DMSO-d₆] 2,1 [3H] s; 5,5 [2H] s; 7,45 [3H] m; 7,7 [2H] d; 7,95 [1H] m; 8,1 [2H] s breit; 8,6 [1H] m; 8,8 [1H] s; 10,2 [1H] s.

### Beispiel 19

### 2-Amino-4-(2,3-dihydro-1,4-benzodioxin-6-yl)-6-[(2-methyl-1,3-thiazol-4-yl)methoxy]-3,5-pyridindicarbonitril

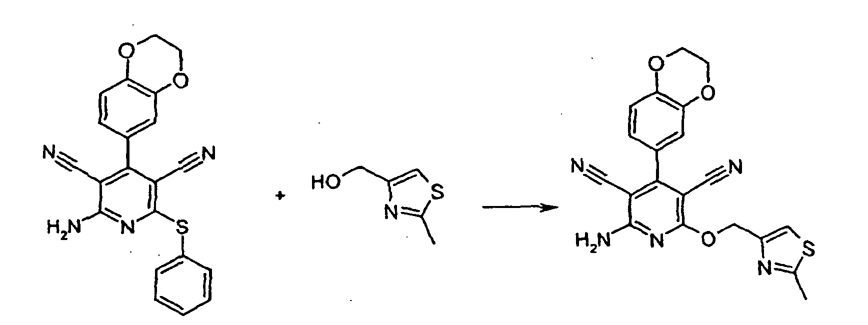

100 mg (0,26 mmol) 2-Amino-4-(2,3-dihydro-1,4-benzodioxin-6-yl)-6-(phenylsulfanyl)-3,5-pyridindicarbonitril [hergestellt analog *Kambe et al.,* Synthesis, 531-533 (1981)] werden zusammen mit 44 mg (0,39 mmol) Kalium tert.butylat und 334 mg (2,59 mmol) (2-Methyl-1,3-thiazol-4-yl)methylalkohol in 5 ml 1,2-Dimethoxyethan über Nacht bei Raumtemperatur gerührt. Nach Eindampfen der Reaktionslösung i.V. wird der Rückstand wird durch präparative HPLC an Reversed Phase Kieselgel gereinigt.
Ausbeute: 41 mg (= 39 % d. Th.)
Massenspektrum: gesuchte Molmasse: 405, gefunden [M+H]⁺= 406
NMR-Spektrum: (¹H-NMR, DMSO-d₆) 2.65 (s, 3H); 4.3 (s, 4H); 5.45 (s, 2H); 7.0 (m, 3H); 7.7 (s, 1H); 8.0 (s breit, 2H).

### Beispiel 20

### 2-Amino-4-(1,3-benzodioxol-5-yl)-6-(3-thienylmethoxy)-3,5-pyridindicarbonitril

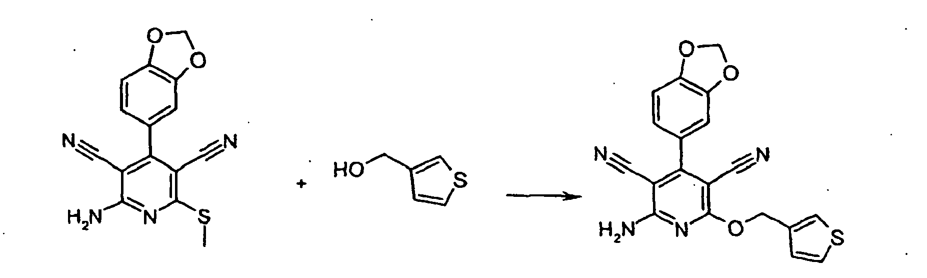

100 mg (0,26 mmol) 2-Amino-4-(2,3-dihydro-1,4-benzodioxin-6-yl)-6-(methylsulfanyl)-3,5-pyridindicarbonitril [hergestellt analog *Dyachenko et al.,* Russian Journal of Chemistry, Vol. 33, No. 7, 1997, Seiten 1014-1017 oder Vol. 34, No. 4, 1998, Seiten 557-563] werden zusammen mit 181 mg (1,6 mmol) Kalium tert.butylat und 184 mg (1,6 mmol) 3-Hydroxymethylthiophen 4 h bei 50°C gerührt. Nach Abkühlen wird die Reaktionsmischung mit Dichlormethan verdünnt und mit Wasser gewaschen. Die organische Phase wird mit Natriumsulfat getrocknet und i.V. eingedampft. Der Rückstand wird aus Diethylether kristallisiert.
Ausbeute: 57 mg (= 47 % d. Th.)
Massenspektrum: gesuchte Molmasse: 376, gefunden [M+H]⁺= 377
NMR-Spektrum: (¹H-NMR, DMSO-d₆) 5.45 (s, 2H); 6.15 (s, 2H); 7.0-7.15 (m, 3H); 7.25 (d, 1H); 7.55 (dd, 1H); 7.7 (d, 1H); 8.0 (s breit, 2H).

## Patentansprüche

1. Verbindungen der Formel (I) 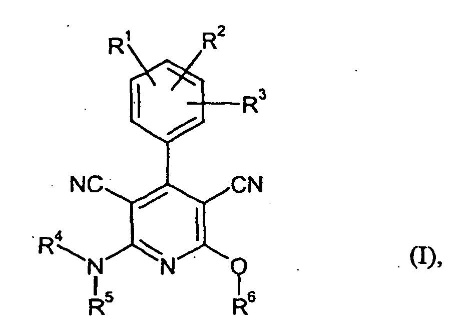 worin
R¹ -NH-C(O)-CH₃ oder -NH-C(O)-C₂H₅ bedeutet und
R² und R³ Wasserstoff bedeuten
oder
R¹ und R² an benachbarte Phenylringatome gebunden sind und für eine Gruppe -O-CH₂-O- oder-O-CH₂-CH₂-O- stehen und
R³ Wasserstoff bedeutet,
R⁴ und R⁵ unabhängig voneinander Wasserstoff, (C₁-C₆)-Alkyl, das durch Hydroxy, (C₁-C₄)-Alkoxy oder Cyclopropyl substituiert sein kann, Cyclopropyl, Benzyl oder Pyridylmethyl bedeuten,
oder
R⁴ und R⁵ gemeinsam mit dem Stickstoffatom, an das sie gebunden sind, einen 5- bis 7-gliedrigen, gesättigten oder partiell ungesättigten Heterocyclus bilden, der ein weiteres Heteroatom aus der Reihe N, O oder S im Ring enthalten kann und der ein- bis dreifach, unabhängig voneinander, durch Hydroxy, (C₁-C₄)-Alkyl oder (C₁-C₄)-Alkoxy substituiert sein kann
und
R⁶ (C₃-C₇)-Cycloalkyl, (C₁-C₆)-Alkyl, das durch (C₃-C₇)-Cycloalkyl, Hydroxy, (C₁-C₄)-Alkoxy, (C₂-C₄)-Alkenyl, Phenyl oder 5- oder 6-gliedriges Heteroaryl mit bis zu drei Heteroatomen aus der Reihe N, O und/oder S substituiert ist, wobei Phenyl und Heteroaryl ihrerseits durch Fluor, Chlor, (C₁-C₄)-Alkyl, (C₁-C₄)-Alkoxy, Amino, Mono- oder Di-(C₁-C₄)-alkylamino, Nitro, Cyano oder Hydroxy substituiert sein können, bedeutet
und ihre Salze, Hydrate, Hydrate der Salze und Solvate.

2. 2-(2-Hydroxyethylamino)-4-(1,3-benzodioxol-5-yl)-6-ethoxy-3,5-pyridindicarbonitril 2-(2-Methoxyethylamino)-4-(1,3-benzodioxol-5-yl)-6-ethoxy-3,5-pydridindicarbonitril 2-Amino-4-phenyl-6-(2-pyridinylmethoxy)-3,5-pydridindicarbonitril 2-Amino-4-phenyl-6-(3-pydridinylmethoxy)-3,5-pydridindicarbonitril 2-Amino-4-phenyl-6-(4-pyridinylmethoxy)-3,5-pyridindicarbonitril 2-Benzyloxy-6-(2-hydroxyethylamino)4-phenyl-3,5-pyridincarbonitril

3. Verfahren zur Herstellung von Verbindungen der Formel (I), wie in Anspruch 1 definiert, **dadurch gekennzeichnet, dass** man entweder
[A] Verbindungen der Formel (II) 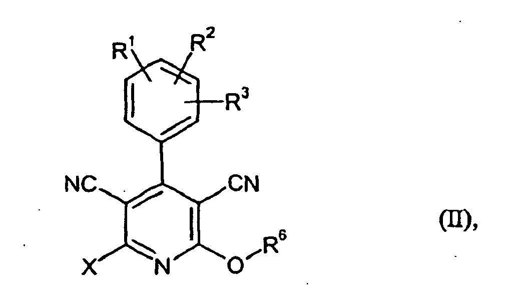 in welcher
R¹, R², R³ und R⁶ die in Anspruch 1 angegebene Bedeutung haben und X für eine Abgangsgruppe steht,
mit Verbindungen der Formel (III)
R⁴ - NH - R⁵ (III),
in welcher
R⁴und R⁵ die in Anspruch 1 angegebene Bedeutung haben,
umsetzt
oder
[B] Verbindungen der Formel (IV) 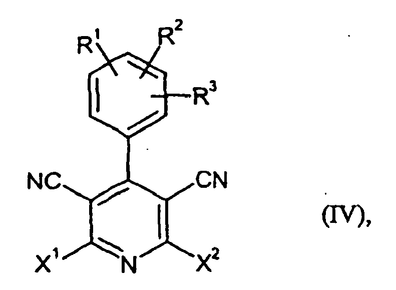 in welcher
R¹, R² und R³ die in Ansprüchen 1 und 2 angegebene Bedeutung haben, und X¹ und X² gleich oder verschieden sind und für Abgangsgruppen stehen,
zunächst mit Verbindungen der Formel (III) in Verbindungen der Formel (V) 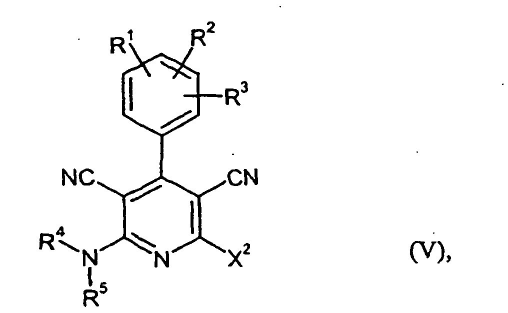 in welcher
R¹, R², R³, R⁴ und R⁵ die in Ansprüchen 1 und 2 angegebene Bedeutung haben und X² für eine Abgangsgruppe steht,
überführt und diese dann mit Verbindungen der Formel (VI)
R⁶ - OH (VI),
in welcher
R⁶ die in Ansprüchen 1 und 2 angegebene Bedeutung hat,
umsetzt
oder
[C] für den Fall, dass in Verbindungen der Formel (I) R⁴ und R⁵ jeweils für Wasserstoff stehen, Verbindungen der Formel (VII) 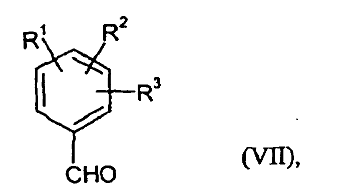 in welcher
R¹, R² und R³ die in Ansprüchen 1 und 2 angegebene Bedeutung haben,
in Gegenwart einer Base mit Malodinitril und Verbindungen der Formel (VI) umsetzt.

4. Verbindungen der Formel (I), wie in Ansprüchen 1 und 2 definiert, zur Prophylaxe und/oder Behandlung von Erkrankungen.

5. Zusammensetzung, enthaltend mindestens eine Verbindungen der Formel (I), wie in Ansprüchen 1 und 2 definiert, und mindestens einen weiteren Hilfsstoff.

6. Verwendung von Verbindungen der Formel (I), wie in Ansprüchen 1 und 2 definiert, zur Herstellung von Arzneimitteln zur Prophylaxe und/oder Behandlung von Erkrankungen des Herzkreislaufsystems (kardiovaskulären Erkrankungen).

7. Verwendung von Verbindungen der Formel (I), wie in Ansprüchen in 1 und 2 definiert, zur Herstellung von Arzneimitteln zur Prophylaxe und/oder Behandlung von Erkrankungen des Urogenitalbereichs und Krebs.

8. Verwendung von Verbindungen der Formel (I), wie in Ansprüchen 1 und 2 definiert, zur Herstellung von Arzneimitteln zur Prophylaxe und/oder Behandlung von inflammatorischen und neuroinflammatorischen Erkrankungen, neurodegenerativen Erkrankungen und Schmerzzuständen.

9. Verwendung von Verbindungen der Formel (I), wie in Ansprüchen 1 und 2 definiert, zur Herstellung von Arzneimitteln zur Prophylaxe und/oder Behandlung von Erkrankungen der Atemwege, von Leberfibrose und Leberzirrhose und Diabetes.

## Claims

1. Compounds of the formula (I) 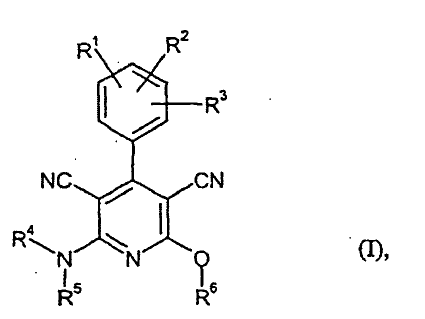 in which
R¹ represents -NH-C(O)-CH₃ or -NH-C(O)-C₂H₅, and
R² and R⁵ represent hydrogen
or
R¹ and R² are attached to adjacent phenyl ring atoms and represent a group -O-CH₂-O- or -O-CH₂-CH₂-O- and
R³ represents hydrogen,
R⁴ and R⁵ independently of one another represent hydrogen, (C₁-C₆)-alkyl which may be substituted by hydroxyl, (C₁-C₄)-alkoxy or cyclopropyl, cyclopropyl, benzyl or pyridylmethyl,
or
R⁴ and R⁵ together with the nitrogen atom to which they are attached form a 5- to 7-membered saturated or partially unsaturated heterocycle which may contain a further heteroatom from the group consisting of N, O or S in the ring and which may be mono- to trisubstituted, independently of one another, by hydroxyl, (C₁-C₄)-alkyl or (C₁-C₄)-alkoxy,
and
R⁶ represents (C₃-C₇)-cycloalkyl or (C₁-C₆)-alkyl, which is substituted by (C₃-C₇)-cycloalkyl, hydroxyl, (C₁-C₄)-alkoxy, (C₂-C₄)-alkenyl, phenyl or 5- or 6-membered heteroaryl having up to three heteroatoms from the group consisting of N, O and/or S, where phenyl and heteroaryl for their part may be substituted by fluorine, chlorine, (C₁-C₄)-alkyl, (C₁-C₄)-alkoxy, amino, mono- or di-(C₁-C₄)-alkylamino, nitro, cyano or hydroxyl,
or a salt, a hydrate, a hydrate of a salt or a solvate thereof.

2. 2-(2-Hydroxyethylamino)-4-(1,3-benzodioxol-5-yl)-6-ethoxy-3,5-pyridinedicarbonitrile
2-(2-methoxyethylamino)-4-(1,3-benzodioxol-5-yl)-6-ethoxy-3,5-pyridinedicarbonitrile
2-amino-4-phenyl-6-(2-pyridinylmethoxy)-3,5-pyridinedicarbonitrile
2-amino-4-phenyl-6-(3-pyridinylmethoxy)-3,5-pyridinedicarbonitrile
2-amino-4-phenyl-6-(4-pyridinylmethoxy)-3,5-pyridinedicarbonitrile
2-benzyloxy-6-(2-hydroxyethylamino)-4-phenyl-3,5-pyridinecarbonitrile.

3. Process for preparing compounds of the formula (I) as defined in Claim 1,
**characterized in that** either
[A] compounds of the formula (II) 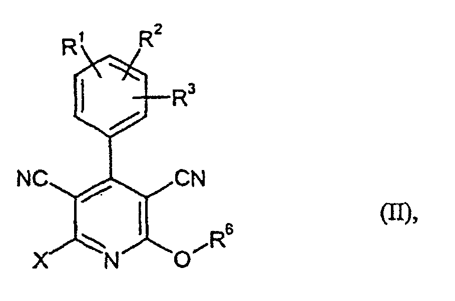 in which
R¹, R², R³ and R⁶ are as defined in Claim 1 and X represents a leaving group,
are reacted with compounds of the formula (III)
R⁴-NH-R⁵ (III)
in which
R⁴ and R⁵ are as defined in Claim 1,
or
[B] compounds of the formula (IV) 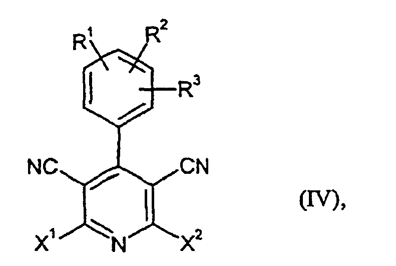 in which
R¹, R² and R³ are as defined in Claims 1 and 2 and X¹ and X² are identical or different and represent leaving groups,
are initially converted with compounds of the formula (III) into compounds of the formula (V) 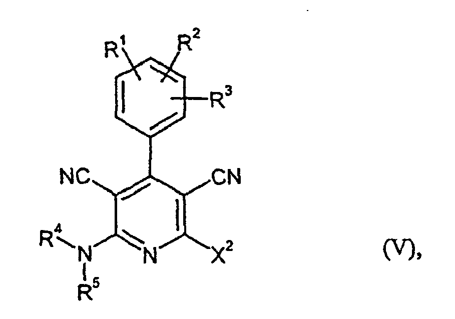 in which
R¹, R², R³, R⁴ and R⁵ are as defined in Claims 1 and 2 and X² represents a leaving group
and these are then reacted with compounds of the formula (VI)
R⁶-OH (VI)
in which
R⁶ is as defined in Claims 1 and 2,
or
[C] if, in compounds of the formula (I), R⁴ and R⁵ each represent hydrogen, compounds of the formula (VII) 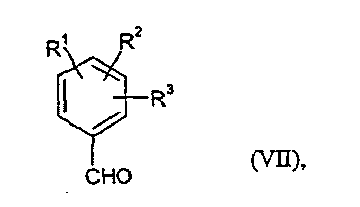
in which
R¹, R² and R³ are as defined in Claims 1 and 2
are reacted in the presence of a base with malonitrile and compounds of the formula (VI).

4. Compounds of the formula (I) as defined in Claims 1 and 2 for the prophylaxis and/or treatment of disorders.

5. Composition, comprising at least one compound of the formula (I) as defined in Claims 1 and 2 and at least one further auxiliary.

6. Use of compounds of the formula (I) as defined in Claims 1 and 2 for preparing medicaments for the prophylaxis and/or treatment of disorders of the cardiovascular system (cardiovascular disorders).

7. Use of compounds of the formula (1) as defined in Claims 1 and 2 for preparing medicaments for the prophylaxis and/or treatment of disorders of the urogenital system and cancer.

8. Use of compounds of the formula (I) as defined in Claims 1 and 2 for preparing medicaments for the prophylaxis and/or treatment of inflammatory and neuroinflammatory disorders, neurodegenerative disorders and pain.

9. Use of compounds of the formula (I) as defined in Claims 1 and 2 for preparing medicaments for the prophylaxis and/or treatment of disorders of the respiratory tract, of liver fibrosis and liver cirrhosis and diabetes.

## Revendications

1. Composés de formule (I) 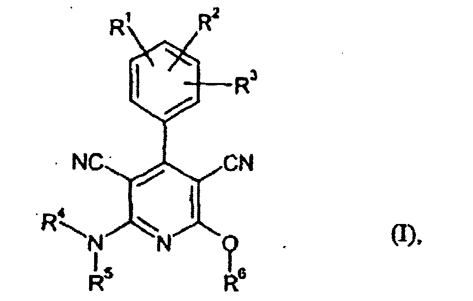 dans laquelle
R¹ représente un groupe -NH-C(O)-CH₃ ou -NH-C (O) -C₂H₅ et
R² et R³ représentent l'hydrogène,
ou bien
R¹ et R² sont liés à des atomes contigus du noyau phényle et représentent un groupe -O-CH₂-O- ou -O-CH₂-CH₂-O- et
R³ représente l'hydrogène,
R⁴ et R⁵ représentent indépendamment l'un de l'autre l'hydrogène, un reste alkyle en C₁ à C₆, qui peut être substitué par un radical hydroxy, alkoxy en C₁ à C₄ ou cyclopropyle, un reste cyclopropyle, benzyle ou pyridylméthyle,
ou bien
R⁴ et R⁵ forment, conjointement avec l'atome d'azote auquel ils sont liés, un hétérocycle pentagonal à heptagonal, saturé ou partiellement insaturé, qui peut contenir un autre hétéroatome de la série N, O ou S dans le noyau ou qui peut porter un à trois substituants hydroxy, alkyle en C₁ à C₄ ou alkoxy en C₁ à C₄ indépendamment les uns des autres
et
R⁶ représente un reste cycloalkyle en C₃ à C₇, alkyle en C₁ à C₆ qui est substitué par un radical cycloalkyle en C₃ à C₇, hydroxy, alkoxy en C₁ à C₄, alcényle en C₂ à C₄, phényle ou hétéroaryle pentagonal ou hexagonal ayant jusqu'à trois hétéroatomes de la série N, O et/ou S, les radicaux phényle et hétéroaryle pouvant eux-mêmes être substitués par un radical fluoro, chloro, alkyle en C₁ à C₄, alkoxy en C₁ à C₄, amino, mono- ou di (alkyle en C₁ à C₄) amino, nitro, cyano ou hydroxy,
et leurs sels, leurs hydrates, les hydrates de leurs sels et leurs produits de solvatation.

2. le 2-(2-hydroxyéthylamino)-4-(1,3-benzodioxol-5-yl)-6-éthoxy-3,5-pyridinedicarbonitrile,
le 2-(2-méthoxyéthylamino)-4-(1,3-benzodioxol-5-yl)-6-éthoxy-3,5-pyridinedicarbonitrile,
le 2-amino-4-phényl-6-(2-pyridinylméthoxy)-3,5-pyridine-dicarbonitrile,
le 2-amino-4-phényl-6-(3-pyridinylméthoxy)-3,5-pyridine-dicarbonitrile,
le 2-amino-4-phényl-6-(4-pyridinylméthoxy)-3,5-pyridine-dicarbonitrile,
le 2-benzyloxy-6-(2-hydroxyéthylamino)-4-phényl-3,5-pyridinecarbonitrile.

3. Procédé de production de composés de formule (I) telle que définie dans la revendication 1, **caractérisé en ce que**
[A] on fait régir des composés de formule (II) 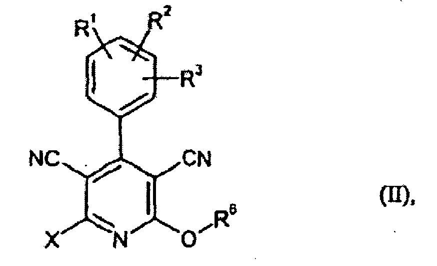 dans laquelle
R¹, R², R³ et R⁶ ont la définition indiquée dans la revendication 1, et X représente un groupe partant,
avec des composés de formule (III)
R⁴-NH-R⁵ (III)
dans laquelle
R⁴ et R⁵ ont la définition indiquée dans la revendication 1,
ou bien
[B] on commence par transformer des composés de formule (IV) 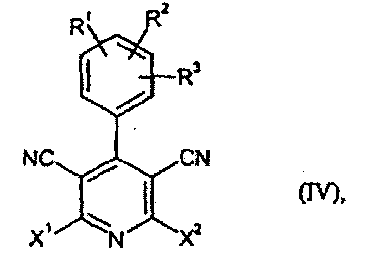 dans laquelle
R¹, R² et R³ ont la définition indiquée dans la revendication 1 ou 2, et X¹ et X² sont identiques ou différents et représentent des groupes partants,
avec des composés de formule (III) en composés de formule (V) 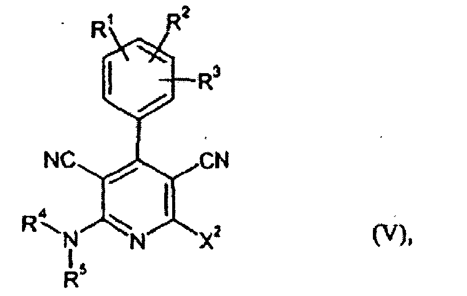 dans laquelle
R¹, R², R³, R⁴ et R⁵ ont la définition indiquée dans les revendications 1 et 2, et X² représente un groupe partant,
puis on fait réagir ces composés avec des composés de formule (VI)
R⁶-OH (VI),
dans laquelle
R⁶ a la définition indiquée dans les revendications 1 et 2,
ou bien
[C] au cas où R⁴ et R⁵ représentent chacun de l'hydrogène dans des composés de formule (I), on fait réagir des composés de formule (VII) 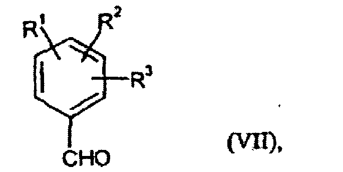 dans laquelle
R¹, R² et R³ ont la définition indiquée dans les revendications 1 et 2.
en présence d'une base, avec le malodinitrile et des composés de formule (VI).

4. Composés de formule (I), telle que définie dans les revendications 1 et 2, destinés à la prophylaxie et/ou au traitement de maladies.

5. Composition contenant au moins un composé de formule (I), telle que définie dans les revendications 1 et 2, et au moins une autre substance active.

6. Utilisation de composés de formule (I), telle que définie dans les revendications 1 et 2, pour la préparation de médicaments destinés à la prophylaxie et/ou au traitement de maladies du système cardio-vasculaire (maladies cardio-vasculaires).

7. Utilisation de composés de formule (I), telle que définie dans les revendications 1 et 2, pour la préparation de médicaments destinés à la prophylaxie et/ou au traitement de maladies des voies urogénitales et du cancer.

8. Utilisation de composés de formule (I), telle que définie dans les revendications 1 et 2, pour la préparation de médicaments destinés à la prophylaxie et/ou au traitement de maladies inflammatoires et neuro-inflammatoires, de maladies neurodégénératives et d'états douloureux.

9. Utilisation de composés de formule (I), telle que définie dans les revendications 1 et 2, pour la préparation de médicaments destinés à la prophylaxie et/ou au traitement de maladies des voies respiratoires, de la fibrose hépatique et de la cirrhose du foie, et du diabète.
